# EUROPEAN PATENT APPLICATION

(11) **EP 4 215 211 A1**
(43) Date of publication of application: **26.07.2023**
(21) Application number: 21863925.0
(22) Date of filing: 21.06.2021
(51) Int. Cl.: A61K 45/00, A61K 31/357, A61K 39/395, A61P 35/00, A61P 37/02, C12Q 1/6844, G01N 33/50, G01N 33/68

(54) **TUMOR IMMUNE MICROENVIRONMENT REGULATOR, AND PREVENTIVE, DIAGNOSTIC OR THERAPEUTIC USE THEREOF**

(30) Priority: 02.09.2020 JP 2020147222
(71) Applicant: The University of Tokyo, Bunkyo-ku, Tokyo 113-8654 (JP)
(72) Inventor: TANIGUCHI Tadatsugu, Tokyo 113-8654 (JP); HANGAI Sho, Tokyo 113-8654 (JP); YANAI Hideyuki, Tokyo 113-8654 (JP)
(74) Representative: Duncombe, Jessica
(86) International application number: PCT/JP2021/023421
(87) International publication number: WO 2022/049867

(57) **Abstract**

The present invention is intended to identify a modulatory substance of the tumor immune microenvironment, and to provide a therapeutic utilization method of the modulatory substance. Specifically, the present invention relates to an inhibitor of the accumulation of myeloid-derived suppressor cells (MDSCs) in the tumor microenvironment (TIME), wherein the inhibitor comprises, an active ingredient, a substance that suppresses or inhibits the function of an immunomodulator released from dead tumor cells. More specifically, the present invention relates to the aforementioned inhibitor comprising a substance that suppresses or inhibits the function of, for example, TCTP (translationally controlled tumor protein).

## Description

### Technical Field

The present invention relates to a modulatory substance of the tumor immune microenvironment, and preventive, diagnostic and therapeutic utilization of the same. More specifically, the present invention relates to an inhibitory substance of the function of TCTP (translationally controlled tumor protein), a drug or a composition for treating cancer, comprising the inhibitory substance, and utilization of the inhibitory substance in the prevention, diagnosis and/or treatment of cancer.

### Background Art

Immune response mechanism against cancer has been discussed for a long period of time, and various study reports have been made. As a result of recent studies, the mechanism thereof, etc. has been finally understood, and the "immunotherapy of cancer" has attracted attention.

At present, main immunotherapies actually used in cancer therapy may include "checkpoint inhibitor antibody therapy" and "CAR-T cell therapy." Regarding the effects of these immunotherapies, it can be confirmed that the immunotherapies exhibit certain effects on certain types of cancers. However, under current circumstances, it cannot be said that these immunotherapies are sufficient also for the treatment of many other cancers.

Both of the above-described two therapies are intended to perform cancer therapy by activation of lymphocytes (that attack cancer). On the other hand, in living bodies, a mechanism of suppressing an immune function that attacks cancer cells, which is referred to as a "tumor immune microenvironment (TIME)," has been known (Non Patent Literature 1 and Non Patent Literature 2). The tumor immune microenvironment is also called a "cradle of cancer," and a mechanism of suppressing an immune response to cancer is present in the tumor immune microenvironment. Thus, it has been known that an environment advantageous for proliferation of cancer cells can be created in the tumor immune microenvironment. As such, it is expected that elucidation of the interaction between cancer cells and immune cells and the interaction between related molecules in TIME will lead to the development of a novel immunotherapy.

By the way, cells that play an important role in the above-described immunosuppressive function associated with progression of tumor may be, for example, myeloid-derived suppressor cells (MDSCs). MDSC is a population of progenitor cells that are present in the bone marrow and each have a different differentiation degree, before being differentiated into granulocytes, dendritic cells, macrophages, etc. Among cell populations constituting MDSCs, in particular, two types of cell populations have been identified in mice and humans, and have attracted attention. One of these two types of cells are polymorphonuclear myeloid-derived suppressor cells (PMN-MDSCs) having phenotypes and morphological features that are similar to those of neutrophils. The other type of cells are monocytic myeloid-derived suppressor cells (M-MDSCs) known to be differentiated into tumor-associated macrophages (TAMs) (Non Patent Literature 3). Regarding the relationship between MDSCs and cancer, it has been reported that, in malignant tumors, the expression level of MDSCs increases and the MDSCs accumulates in TIME, and that the MDSCs show a direct correlation with clinical cancer stage, the amount of metastatic tumor, and the prognosis of cancer. Since MDSCs actually suppress the activation and/or proliferation of lymphocytes (Non Patent Literature 4), it is conceived that the MDSCs play a role in promoting progression of tumor.

However, the mechanism of accumulation of MDSCs in TIME has not yet been elucidated.

### Citation List

### Non Patent Literature

Non Patent Literature 1: Binnewies et al., Nature medicine 24, 541-550, doi: 10.103 8/s41591-018-0014-x (2018).
Non Patent Literature 2: Munn et al., Current opinion in immunology 39, 1-6, doi: 10.1016/j.coi.2015.10.009 (2016).
Non Patent Literature 3: Kumar et al., Trends in immunology 37, 208-220, doi: 10.1016/j.it.2016.01.004 (2016).
Non Patent Literature 4: Fleming et al., Frontiers in immunology 9, 398, doi: 10.3389/fimmu.2018.00398 (2018).
Non Patent Literature 5: Hangai et al., Proceedings of the National Academy of Sciences of the United States of America 113, 3844-3849, doi: 10.1073/pnas.1602023113 (2016).
Non Patent Literature 6: Katoh et al., Cancer cell 24, 631-644, doi: 10.1016/j.ccr.2013.10.009 (2013).
Non Patent Literature 7: Hsu et al., Nature 445, 785-788, doi: 10.1038/nature05528 (2007).
Non Patent Literature 8: Cans et al., Proceedings of the National Academy of Sciences of the United States of America 100, 13892-13897, doi: 10.1073/pnas.2335950100 (2003).
Non Patent Literature 9: Amson et al., Nature medicine 18, 91-99, doi: 10.1038/nm.2546 (2011).
Non Patent Literature 10: Gong et al., Nature reviews. Immunology 20, 95-112, 250 doi: 10.1038/s41577-019-0215-7 (2020).
Non Patent Literature 11: Fujita et al., FEBS letters 582 1055-1060, doi: 10.1016/j.febslet.2008.02.055 (2008).
Non Patent Literature 12: Hiraoka et al., British journal of cancer. 103, 1057-1065, (2010).
Non Patent Literature 13: Marjou et al., Genesis. 39(3), 186-93, (2004).
Non Patent Literature 14: Zhang et al., The Journal of Gene Medicine. 7(3), 354-65, (2005).
Non Patent Literature 15: Schroder et al., Scientific Reports. 8(1), 13399, (2018).

### Summary of Invention

### Technical Problem

Considering the aforementioned circumstances, it is an object of the present invention to identify a modulatory substance of the tumor immune microenvironment and to provide a therapeutic utilization method involving inhibition by the modulatory substance.

### Solution to Problem

It has been known that a majority of tumor cells die during the proliferation thereof due to necrosis as a main cause, as a result of mutagenesis or changes in the surrounding environment (for example, hypoxia, etc.) (Non Patent Literature 12). The present inventors have made a working hypothesis that molecules released from dead (which hereinafter means "necrotic") tumor cells (hereinafter also referred to as "dead tumor cells") function as immunomodulators of MDSCs in TIME, and the present inventors have then conducted intensive studies regarding the working hypothesis. As a result, the present inventors have discovered for the first time that a translationally controlled tumor protein (hereinafter referred to as "TCTP") released from deal tumor cells is a novel immunomodulator that controls the functions and/or dynamics of MDSCs in TIME.

Specifically, the present inventors have clarified that extracellular TCTP mainly acts on M-MDSCs, so that it induces the expression of a CXCL1 family chemokine. The CXCL1 family chemokine induces PMN-MDSCs to TIME, so that the TIME becomes a highly immunosuppressive state. The present inventors have clarified that induction of PMN-MDSCs to TIME is inhibited by administration of an anti-TCTP monoclonal antibody or a TCTP function inhibitory substance, and that proliferation of tumors is thereby suppressed. The present invention have overcome an unsolved problem regarding how the tumor constructs and controls TIME, by identification of TCTP and clarification of a novel function thereof, and then, have developed a method for inhibiting TCTP, thereby completing the present invention.

The present invention includes the following (1) to (13).
(1) An inhibitor of the accumulation of myeloid-derived suppressor cells (MDSCs) in the tumor microenvironment (TIME), wherein the inhibitor comprises, an active ingredient, a substance that suppresses or inhibits the function of an immunomodulator released from dead tumor cells.
(2) The inhibitor according to the above (1), wherein the myeloid-derived suppressor cells are polymorphonuclear myeloid-derived suppressor cells (PMN-MDSCs).
(3) The inhibitor according to the above (1) or (2), wherein the immunomodulator is TCTP (translationally controlled tumor protein).
(4) The inhibitor according to the above (3), wherein the substance that suppresses or inhibits the function of TCTP is an antibody.
(5) The inhibitor according to the above (3), wherein the substance that suppresses or inhibits the function of TCTP is dihydroartemisinin (DHA).
(6) A therapeutic drug or composition for cancer, comprising, as an active ingredient, the inhibitor according to any one of the above (1) to (5).
(7) The therapeutic drug or composition according to the above (6), wherein the cancer is colorectal cancer, malignant melanoma, or fibrosarcoma.
(8) A method for diagnosing or auxiliarily diagnosing cancer, comprising measuring the amount of TCTP mRNA or TCTP protein that is present in a sample derived from a subject.
(9) The method according to the above (8), wherein the sample is blood or tissue.
(10) An antibody, which is characterized in that the amino acid sequences of CDRs (complementarity determining regions) 1 to 3 satisfy any of the following (A), (B) or (C), or an antigen-binding fragment thereof:
   (A) the CDRs have:
      heavy chain CDR1 comprising the amino acid sequence as set forth in SEQ ID NO: 1,
      heavy chain CDR2 comprising the amino acid sequence as set forth in SEQ ID NO: 2,
      heavy chain CDR3 comprising the amino acid sequence as set forth in SEQ ID NO: 3,
      light chain CDR1 comprising the amino acid sequence as set forth in SEQ ID NO: 4,
      light chain CDR2 comprising the amino acid sequence as set forth in SEQ ID NO: 5,
         and
      light chain CDR3 comprising the amino acid sequence as set forth in SEQ ID NO: 6,
   (B) the CDRs have:
      heavy chain CDR1 comprising the amino acid sequence as set forth in SEQ ID NO: 7,
      heavy chain CDR2 comprising the amino acid sequence as set forth in SEQ ID NO: 8,
      heavy chain CDR3 comprising the amino acid sequence as set forth in SEQ ID NO: 9,
      light chain CDR1 comprising the amino acid sequence as set forth in SEQ ID NO: 10,
      light chain CDR2 comprising the amino acid sequence as set forth in SEQ ID NO: 11,
         and
      light chain CDR3 comprising the amino acid sequence as set forth in SEQ ID NO: 12,
      or
   (C) the CDRs have:
      heavy chain CDR1 comprising the amino acid sequence as set forth in SEQ ID NO: 13,
      heavy chain CDR2 comprising the amino acid sequence as set forth in SEQ ID NO: 14,
      heavy chain CDR3 comprising the amino acid sequence as set forth in SEQ ID NO: 15,
      light chain CDR1 comprising the amino acid sequence as set forth in SEQ ID NO: 16,
      light chain CDR2 comprising the amino acid sequence as set forth in SEQ ID NO: 17,
         and
      light chain CDR3 comprising the amino acid sequence as set forth in SEQ ID NO: 18.

(11) An antibody that suppresses or inhibits the function of TCTP, wherein the antibody competitively inhibits the binding of the antibody according to the above (10) to TCTP, or an antigen-binding fragment thereof.

(12) The antibody according to the above (10) or (11), which is characterized in that it is a humanized antibody, or an antigen-binding fragment thereof.

(13) The antigen-binding fragment according to any one of the above (10) to (12), which is characterized in that it is Fab, Fab', F(ab')₂, Fv, a single chain antibody, scFv, an scFv dimer, or dsFv.

It is to be noted that the symbol "-" ("to") means a numerical range including the values located right and left of the symbol.

### Advantageous Effects of Invention

According to the present invention, a novel therapeutic agent for cancer and a novel method for treating cancer are provided.

### Brief Description of the Drawings

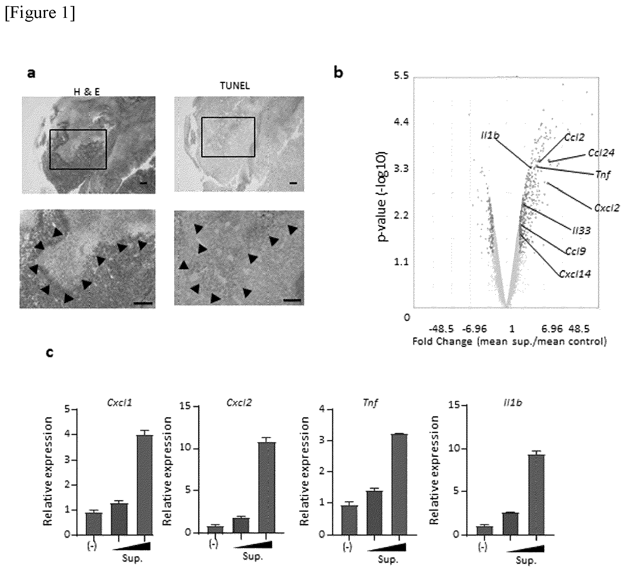

[Figure 1] Figure 1 shows studies regarding the influence of molecules released from dead tumor cells on immune cells. Figure 1(a) shows representative examples of SL4 cells stained by hematoxylin/eosin (H & E) staining and TUNEL staining. The lower view is an enlarged image of the inside of the framework of the upper view. The arrowhead indicates a necrotic lesion. The scale bar indicates 100 µm. In Figure 1(b), PECs (2 × 10⁵ cells) were stimulated with a supernatant of dead SL4 cells (2 × 10⁶ cells) for 2 hours, and a fluctuation in the gene expression in the PECs was then analyzed using a microarray (n = 2). The figure shows a Volcano plot, in which a gene whose expression is fluctuated is indicated with red or green. Figure 1(c) shows the results obtained by stimulating PECs (2 × 10⁵ cells) with a supernatant of dead SL4 cells (2 × 10⁶ cells) for 2 hours, and then quantifying the expressed Cxcl1 mRNA, Cxcl2 mRNA, Tnf mRNA, and Il1b mRNA according to RT-qPCR (n = 3). The data are shown as a mean value ± standard deviation (s.e.m.).

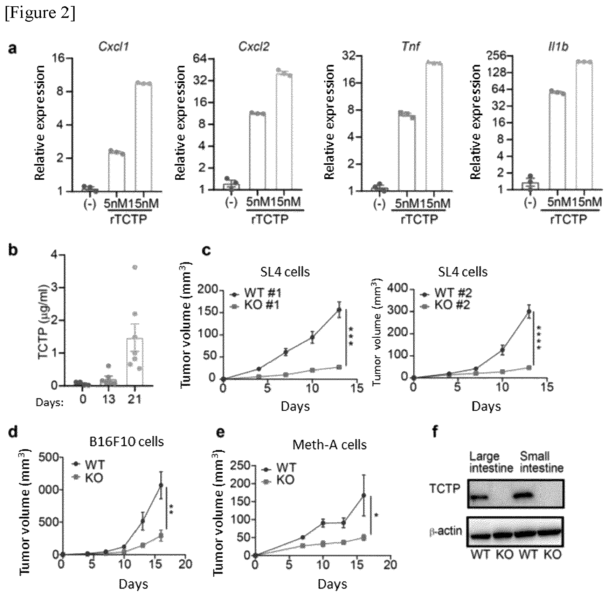

[Figure 2] Figure 2 shows studies regarding the influence of TCTP on tumor proliferation (1). Figure 2(a) shows the results obtained by stimulating PECs (2 × 10⁵ cells) with 5 nM or 15 nM recombinant TCTP for 2 hours, and then quantifying the expressed Cxcl1 mRNA, Cxcl2 mRNA, Tnf mRNA, and Il1b mRNA according to RT-qPCR (n = 3). In Figure 2(b), the sera of non-tumor bearing mice (NTB; n = 4) and mice bearing SL4 cell tumors (Day 0, n = 4) were recovered on Day 13 (n = 6) and Day 21 (n = 7) after transplantation of the tumors, and the amount of TCTP in the sera was then quantified by immunoblotting. In Figure 2(c), TCTP WT SL4 cells (TCTP-expressing SL4 cells) and TCTP KO SL4 cells (TCTP-deficient SL4 cells) (2 × 10⁵ cells each) were transplanted into C57BL/6 mice via subcutaneous injection (WT #1 and KO #1: n = 4; WT #2: n = 6; and KO #2: n = 5). Thereafter, the volume of the tumor was measured over time. In Figure 2(d), TCTP WT B16F10 cells (TCTP-expressing B16F10 cells) and TCTP KO B16F10 cells (TCTP-deficient B16F10 cells) (1 × 10⁵ cells each) were transplanted into C57BL/6 mice via subcutaneous injection (n = 7). Thereafter, the volume of the tumor was measured over time. In Figure 2(e), TCTP WT Meth-A-induced sarcoma cells (hereinafter referred to as "Meth-A cells") and TCTP KO Meth-A cells (1 × 10⁵ cells) were transplanted into C57BL/6 mice via subcutaneous injection (n = 7). Thereafter, the volume of the tumor was measured over time. In Figure 2(f), immunoblotting was carried out on TCTP protein and β-actin present in the intestinal epithelial cells of tamoxifen-treated TCTP^{flox/flox}, Apc^{+/Δ716},villin-Cre ERT2 mice (KO) or tamoxifen non-treated mice (WT). Regarding Figure 2(c) to (e), *P < 0.05, **P < 0.01, ***P < 0.001, ****P < 0.0001, and unpaired two-sided Student's t-test. NS indicates "no significant difference." The data are shown as a mean value ± standard deviation (s.e.m.).

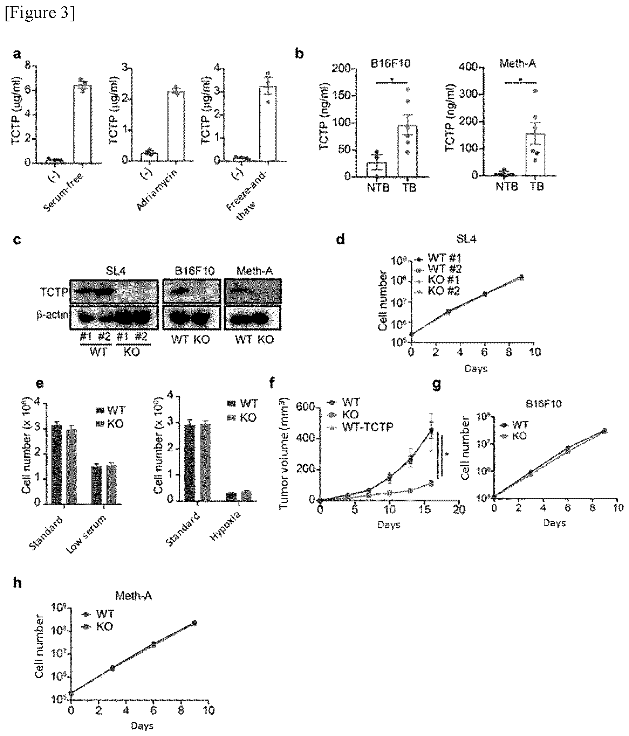

[Figure 3] Figure 3 shows studies regarding the influence of TCTP on tumor proliferation (2). Figure 3(a) shows that apoptosis (serum-free and adriamycin) or necrosis (freeze-and-thaw) was induced to SL4 cells. A culture supernatant was recovered, and the amount of the TCTP protein was then measured by immunoblotting (n = 3). In Figure 3(b), the sera derived from mice bearing B16F10 tumor (left) or Meth-A tumor (right) were recovered on Day 0 (NTB; n = 3) and Day 21 (NTB; n = 3) after subcutaneous transplantation of the tumor cells, and the amount of the TCTP protein was then measured by immunoblotting. In Figure 3(c), the expression of the TCTP protein in wild-type (WT) or TCTP gene inactive-type SL4 cells, B16F10 cells, and Meth-A cells was examined. The whole cell lysate of each cell line was prepared, and an immunoblotting analysis was then carried out regarding TCTP and β-actin. Figure 3(d) shows changes over time in the numbers of TCTP WT SL4 cells and TCTP KO SL4 cells, which were allowed to proliferate *in vitro* (n = 3). In Figure 3(e), TCTP WT SL4 cells and TCTP KO SL4 cells (2 × 10⁵ cells) were cultured under normal (20% O₂, 10% FBS), low serum (0% O₂, 1% FBS) or hypoxic (1% O₂, 10% FBS) condition. Seventy-two hours after the culture, cell numbers were counted. In Figure 3(f), TCTP WT SL4 cells, TCTP KO SL4 cells, and TCTP cDNA-introduced TCTP KO SL4 cells (TCTP transduced) (2 × 10⁵ cells each) were subcutaneously transplanted into C57BL/6 mice (n = 5), and the volumes of the generated tumors were then measured over time. Figure 3(g) shows changes over time in the numbers of TCTP WT Meth-A cells and TCTP KO Meth-A cells, which were allowed to proliferate *in vitro* (n = 3). Figure 3(h) shows changes over time in the numbers of TCTP WT B16F10 cells and TCTP KO B16F10 cells, which were allowed to proliferate *in vitro* (n = 3). Regarding Figure 3(b), *P < 0.05, and unpaired two-sided Student's t-test. Regarding Figure 3(f), Repeated measures one-way ANOVA with Tukey's multiple comparisons test. The data are shown as a mean value ± standard deviation (s.e.m.).

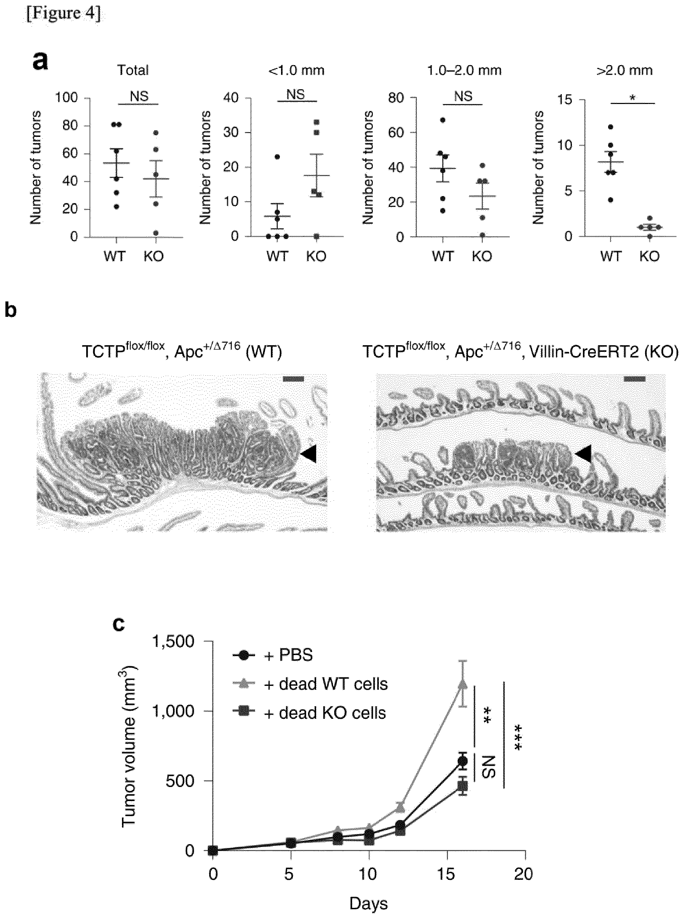

[Figure 4] Figure 4 shows studies regarding the influence of TCTP on tumor proliferation (3). Figure 4(a) shows the total number of intestinal polyps, the number of intestinal polyps having a diameter of less than 1.0 mm, the number of intestinal polyps having a diameter of 1.0 to 2.0 mm, and the number of intestinal polyps having a diameter of more than 2.0 mm, which were developed in 10-week-old TCTP^{flox/flox}, Apc^{+/Δ716} mice (WT; n = 6) or TCTP^{flox/flox}, Apc^{+/Δ716}, villin-Cre ERT2 mice (KO; n = 5). Figure 4(b) shows representative examples of intestinal polyps developed in 10-week-old TCTP^{flox/flox}, Apc^{+/Δ716} mice (WT) or TCTP^{flox/flox}, Apc^{+/Δ716}, villin-Cre ERT2 mice (KO) stained by hematoxylin/eosin staining. The scale bar indicates 200 µm. The arrowhead indicates a tumor. In Figure 4(c), a lethal amount (100 Gy) of X-ray was applied to TCTP WT cells or TCTP KO cells, and the resulting cells were then mixed with TCTP WT (2 × 10⁵ cells). The obtained cell mixture was transplanted into the subcutis of C57BL/6 mice (n = 6). The tumor volume was measured over time. Regarding Figure 4(a), *P < 0.05, **P < 0.01, ***P < 0.001, ****P < 0.0001, and unpaired two-sided Student's t-test. Regarding Figure 4(c), repeated measures one-way ANOVA with Tukey's multiple comparisons test. NS indicates "no significant difference." The data are shown as a mean value ± standard deviation (s.e.m.).

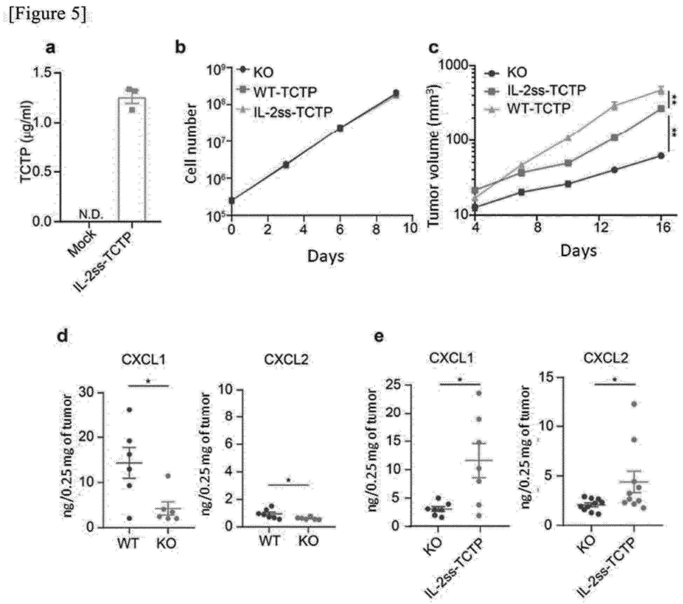

[Figure 5] Figure 5 shows studies regarding the function of extracellular TCTP (1). In Figure 5(a), the culture supernatants of TCTP KO SL4 cells (Mock) and IL-2ss-TCTP SL4 cells (IL-2ss-TCTP) were recovered, and the amount of the TCTP protein was then measured by immunoblotting. (n = 3). In Figure 5(b), the numbers of TCTP KO SL4 cells, IL-2ss-TCTP SL4 cells, and WT-TCTP SL4 cells during the culture were counted over time (n = 4). In Figure 5(c), TCTP KO SL4 cells, IL-2ss-TCTP SL4 cells, or WT-TCTP SL4 cells (2 × 10⁵ cells) were subcutaneously transplanted into C57BL/6 mice (n = 4), and the tumor volume was then measured over time. In Figure 5(d), WT-TCTP SL4 cells or TCTP KO SL4 cells (2 × 10⁵ cells) were subcutaneously transplanted into C57BL/6 mice (n = 4). On Day 21 after the transplantation, the tumor was excised, and the whole tumor lysate was then prepared. The protein amounts of CXCL1 (left, n = 6) and CXCL2 (right, WT: n = 7, KO: n = 6) in the prepared lysate were measured according to ELISA. In Figure 5(e), TCTP KO SL4 cells (Mock) or IL-2ss-TCTP SL4 cells (2 × 10⁵ cells) were subcutaneously transplanted into C57BL/6 mice. On Day 21 after the transplantation, the tumor was excised, and the whole tumor lysate was then prepared. The protein amounts of CXCL1 (left, n = 7) and CXCL2 (right, n = 10) in the prepared lysate were measured according to ELISA. Regarding Figure 5(c), *P < 0.05, **P < 0.01, and repeated measures one-way ANOVA with Dunnett's multiple comparisons test. Regarding Figures 5(d) and (e), unpaired two-sided Student's t-test. ND indicates "not detected." The data are shown as a mean value ± standard deviation (s.e.m.).

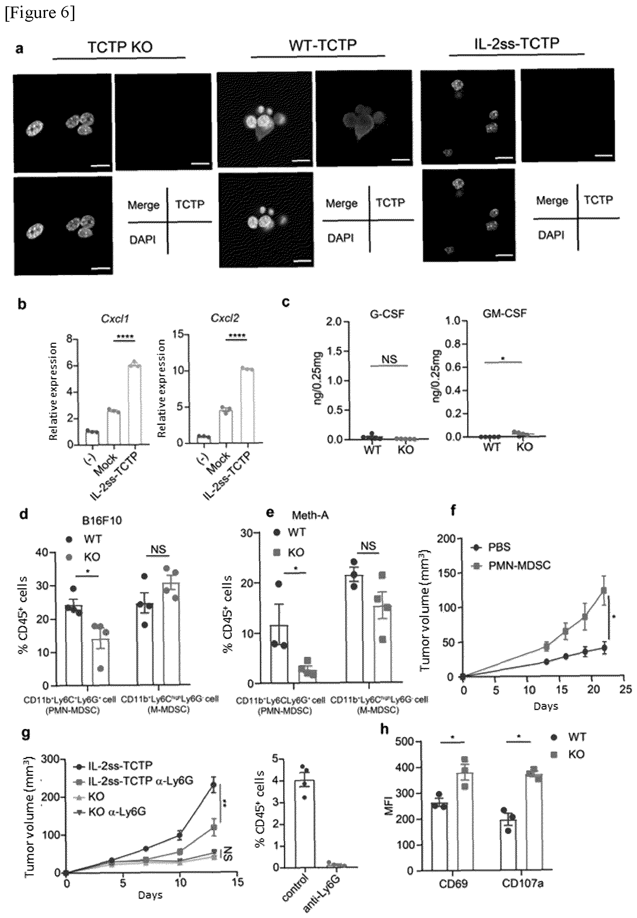

[Figure 6] Figure 6 shows studies regarding the function of extracellular TCTP (2). In Figure 6(a), the TCTP and nucleus (DAPI) of TCTP KO SL4 cells (left view), WT-TCTP cells (TCTP KO cells expressing the cDNA of WT TCTP; center view), and IL-2ss-TCTP SL4 cells (TCTP secretory cells expressing the cDNA of IL-2ss-TCTP; right view) were stained. In Figure 6(b), PECs (2 × 10⁵ cells) were stimulated with a culture supernatant of TCTP KO SL4 cells (Mock) or a culture supernatant of IL-2SS-TCTP SL4 cells (IL2ss-TCTP) for 2 hours. Thereafter, the expressed Cxcl1 and Cxcl2 mRNA were quantified by RT-qPCR (n = 3). In Figure 6(c), WT SL4 cells or TCTP KO SL4 cells (2 × 10⁵ cells) were subcutaneously transplanted into C57BL/6 mice. On Day 21 after the transplantation, the tumor was excised, and the whole tumor lysate was then prepared. The amounts of G-CSF (left) and GM-CSF (right) proteins in the prepared lysate were measured using a cytometric bead assay (CBA). In Figure 6(d), TCTP WT B16F10 cells (WT) or TCTP KO B16F10 cells (KO) were subcutaneously transplanted into C57BL/6 mice. On Day 17 after the transplantation, single cell suspensions were prepared from tumors, and were then subject to a flow cytometry analysis (n = 4). The abundance ratios of PMN-MDSCs or M-MDSCs in the CD45⁺ cell population were indicated with %. In Figure 6(e), TCTP WT Meth-A cells (WT; n = 3) and TCTP KO Meth-A cells (KO; n = 4) were subcutaneously transplanted into C57BL/6 mice. On Day 20 after the transplantation, single cell suspensions were prepared from tumors, and were then subject to a flow cytometry analysis. The abundance ratios of PMN-MDSCs or M-MDSCs in the CD45⁺ cell population were indicated with %. In Figure 6(f), TCTP KO SL4 cells were subcutaneously transplanted into C57BL/6 mice. On Days 1, 4, 7, 10 and 13 after the transplantation, polymorphonuclear myeloid-derived suppressor cells derived from the spleen and bone marrow of mice bearing SL4 tumors (hereinafter referred to as "PMN-MDSCs") or PBS were intraperitoneally injected into the mice. The tumor volume was measured over time. PBS: n = 5; and PMN-MDSC: n = 4. In Figure 6(g), TCTP KO SL4 cells and IL-2ss-TCTP SL4 cells (2 × 10⁵ cells) were each subcutaneously transplanted into C57BL/6 mice. An anti-Ly6G antibody or control IgG was intraperitoneally administered to the mice every three days (n = 5). (Left view) The tumor volume was measured over time. (Right view) The abundance ratio of PMN-MDSCs in the CD45⁺ cell population was indicated with %. In Figure 6(h), TCTP WT SL4 cells (WT) and TCTP KO SL4 cells (KO) were subcutaneously transplanted into C57BL/6 mice (n = 3). On Day 18 after the transplantation, single cell suspensions were prepared from tumors, and were then subject to a flow cytometry analysis. The mean fluorescence intensity (MFI) of markers serving as indicators for activation of NK cells (CD69 and CD107a) was determined. Regarding Figures 6(b) to (f) and (h), *P < 0.05, *P < 0.01, ****P < 0.0001, and unpaired two-sided Student's t-test. Regarding Figure 6(g), repeated measures one-way ANOVA with Tukey's multiple comparisons test. NS indicates "no significant difference." The data are shown as a mean value ± standard deviation (s.e.m.).

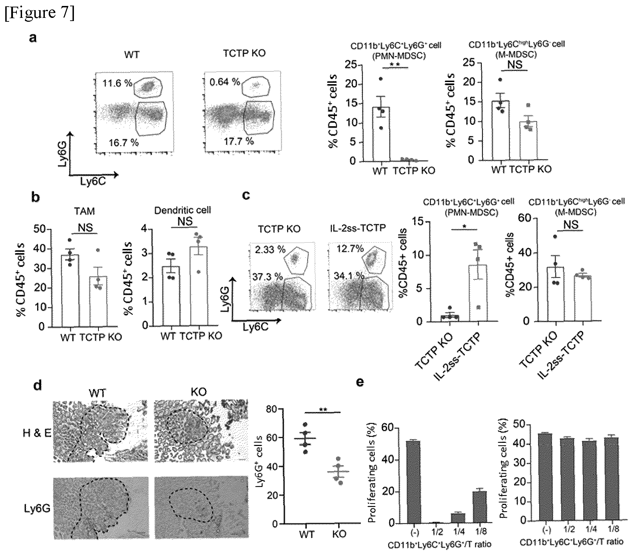

[Figure 7] Figure 7 shows studies regarding the immunosuppressive function of TCTP in TIME. In Figure 7(a) and (b), WT SL4 cells or TCTP KO SL4 cells (2 × 10⁵ cells) were subcutaneously transplanted into C57BL/6 mice. On Day 19 after the transplantation, single cell suspensions were prepared from tumors, and were then subject to a flow cytometry analysis (n = 4). In Figure 7(a), (left view) representative plots, and (right view) the abundance ratios of PMN-MDSCs (CD11b⁺Ly6C⁺Ly6G⁺ cells) and M-MDSCs (CD11b⁺Ly6C^{high} Ly6G⁻ cells) in the CD45⁺ cell population, which were indicated with %. In Figure 7(b), the abundance ratio of TAMs (CD11b⁺F4/80⁺ cells) and DCs (CD11b⁻CD11c⁺ cells) in the CD45⁺ cell population was indicated with %. In Figure 7(c), TCTP KO SL4 cells or IL-2ss-TCTP SL4 cells (2 × 10⁵ cells) were subcutaneously transplanted into C57BL/6 mice. On Day 19 after the transplantation, single cell suspensions were prepared from tumors, and were then subject to a flow cytometry analysis (n = 4). (a) (left view) representative plots, and (right view) the abundance ratios of PMN-MDSCs and M-MDSCs in the CD45⁺ cell population, which were indicated with %. In Figure 7(d), (left view) representative examples of colorectal cancer tissues derived from TCTP^{flox/flox}, Apc^{+/Δ716} mice (WT), and TCTP^{flox/flox}, Apc^{+/Δ716}, Villin-CreERT2 mice (KO), which were stained by hematoxylin/eosin (H & E) staining and Ly6G staining. The scale indicates 100 µm. In Figure 7(d), the right view shows the results obtained by quantifying the number of Ly6G⁺ cells present in a region of 90,000 µm² in the visual fields of WT tumor tissues and KO tumor tissues (n = 4). The region surrounded by the broken line indicates a tumor. Figure 7(e) shows the effect of CD11b⁺Ly6C⁺Ly6G⁺ cells separated from the spleen of mice bearing SL4 tumors to suppress proliferation of T cells. T cells were stimulated with anti-CD3/CD28 in the presence of CD11b⁺Ly6C⁺Ly6G⁺ cells derived from mice bearing tumors (left) or non-tumor bearing mice (right). The ratio of proliferating T cells was determined by dilution of CFSE (carboxyfluorescein diacetate succinimidyl ester) (n = 3). Regarding Figures 7(a) to (d), *P < 0.05, *P < 0.01, ****P < 0.0001, and unpaired two-sided Student's t-test. NS indicates "no significant difference." The data are shown as a mean value ± standard deviation (s.e.m.).

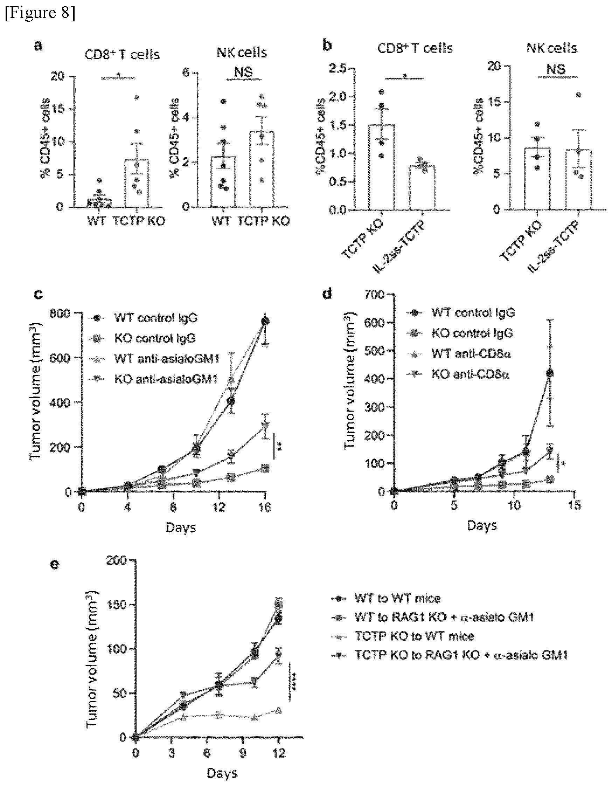

[Figure 8] Figure 8 shows studies regarding the influence of TCTP on the numbers of T cells and NK cells, and the antitumor activity thereof. In Figure 8(a), WT SL4 cells or TCTP KO SL4 cells (2 × 10⁵ cells) were subcutaneously transplanted into C57BL/6 mice. On Day 19 after the transplantation, single cell suspensions were prepared from tumors, and were then subjected to a flow cytometry analysis (WT: n = 7, KO: n = 6). The abundance ratio of CD8⁺ T cells (CD3ε⁺NK1.1⁻CD8⁺ cells) and NK cells (CD3ε⁻NK1.1⁺ cells) in the CD45⁺ cell population was indicated with %. In Figure 8(b), a Mock-IRES-GFP vector (Mock) or an IL-2ss-TCTP-IRES-GFP vector (IL-2ss-TCTP) was introduced into TCTP KO cells. Thereafter, GFP-positive cells were separated by cell sorting. Mock or IL-2ss-TCTP cells (2 × 10⁵ cells) were subcutaneously transplanted into C57BL/6 mice. On Day 19 after the transplantation, single cell suspensions were prepared from tumors, and were then subject to a flow cytometry analysis (n = 4). The abundance ratio of CD8⁺ T cells (CD3ε⁺NK1.1⁻CD8⁺ cells) and NK cells (CD3ε⁻NK1.1⁺ cells) in the CD45⁺ cell population was indicated with %. In Figures 8(c) and (d), WT SL4 cells or TCTP KO SL4 cells (2 × 10⁵ cells) were subcutaneously transplanted into C57BL/6 mice. In Figure 8(c), an anti-Asialo GM1 antibody (WT: n = 4, KO: n = 6) or control IgG (WT: n = 5, KO: n = 6) was intraperitoneally administered to the mice on one day before the transplantation and on Days 3, 7, 11, and 15 after the transplantation. In Figure 8(d), anti-D8α (n = 5) or control IgG (n = 6) was intraperitoneally administered to the mice on one day before the transplantation and on Days 2, 5, 8, and 11 after the transplantation. In Figure 8(e), WT SL4 cells or TCTP KO SL4 cells (2 × 10⁵ cells) were subcutaneously transplanted into WT mice (WT: n = 5, KO: n = 6) or RAG1 KO mice (n = 3). An anti-Asialo GM1 antibody or control IgG was intraperitoneally administered to the mice on one day before the transplantation and on Days 3, 7, and 11 after the transplantation. Regarding Figures 8(a) to (e), *P < 0.05, ****P < 0.0001, and unpaired two-sided Student's t-test. The data are shown as a mean value ± standard deviation (s.e.m.).

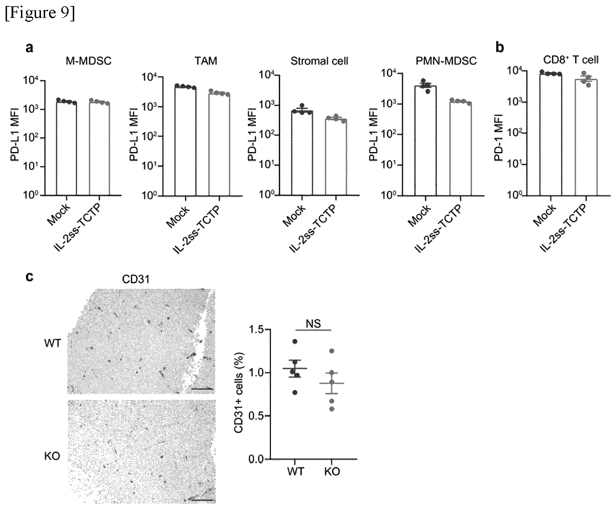

[Figure 9] Figure 9 shows studies regarding the influence of TCTP on the expression of immune checkpoint molecules and angiogenesis. In Figures 9(a) and (b), WT SL4 cells and TCTP KO SL4 cells (2 × 10⁵ cells) were subcutaneously transplanted into C57BL/6 mice. On Day 21 after the transplantation, single cell suspensions were prepared from tumors, and were then subject to a flow cytometry analysis. The mean fluorescence intensity (MFI) of PD-L1 (a) and PD-1 (b) was determined. In Figure 9(c), (left view) representative examples of the CD31 stained images of a TCTP WT SL4 tumor or a TCTP KO SL4 tumor, which was subcutaneously transplanted into C57BL/6. The scale indicates 100 µm. In Figure 9(c), (right view) quantification of a CD31-positive area. NS indicates "no significant difference." Regarding Figure 9(c), unpaired two-sided Student's t-test. The data are shown as a mean value ± standard deviation (s.e.m.).

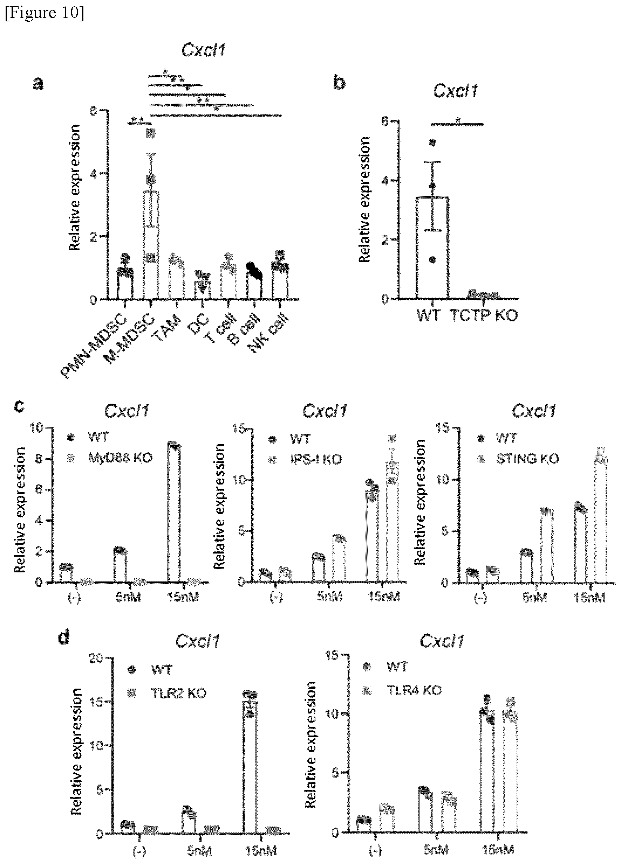

[Figure 10] Figure 10 shows identification of cells targeted by TCTP and TCTP receptor (1). In Figure 10(a), SL4 cells (2 × 10⁵ cells) were subcutaneously transplanted into C57BL/6 mice (n = 3). On Day 21 after the transplantation, single cell suspensions were prepared from tumors. Various types of immune cells (PMN-MDSCs, M-MDSCs, TAMs, DCs, T cells, B cells, and NK cells) were separated, and the Cxcl1 mRNA of individual cells was then quantified by qRT-PCR. In Figure 10(b), TCTP WT SL4 cells (2 × 10⁵ cells) and TCTP KO SL4 cells (2 × 10⁵ cells) were subcutaneously transplanted into C57BL/6 mice (n = 3). On Day 21 after the transplantation, single cell suspensions were prepared from tumors. Thereafter, M-MDSCs was separated, and the Cxcl1 mRNA of individual cells was then quantified by qRT-PCR. In Figure 10(c), peritoneal exudate cells (hereinafter referred to as "PECs") derived from mice having various genotypes (WT (wild type), MyD88 KO (MyD88-deficient type), IPS-I KO (IPS-I-deficient type), and STING KO (STING-deficient type)) were stimulated with recombinant TCTP. Cxcl1 mRNA was quantified by qRT-PCR (n = 3). In Figure 10(d), PECs derived from mice having various genotypes (WT, TLR2 KO (TLR2-deficient type), and TLR4 KO (TLR4-deficient type)) were stimulated with recombinant TCTP. Cxcl1 mRNA was quantified by qRT-PCR (n = 3). Regarding Figure 10(b), *P < 0.05, **P < 0.01, and unpaired two-sided Student's t-test. Regarding Figure 10(a), repeated measures one-way ANOVA with Dunnett's multiple comparisons test. The data are shown as a mean value ± standard deviation (s.e.m.).

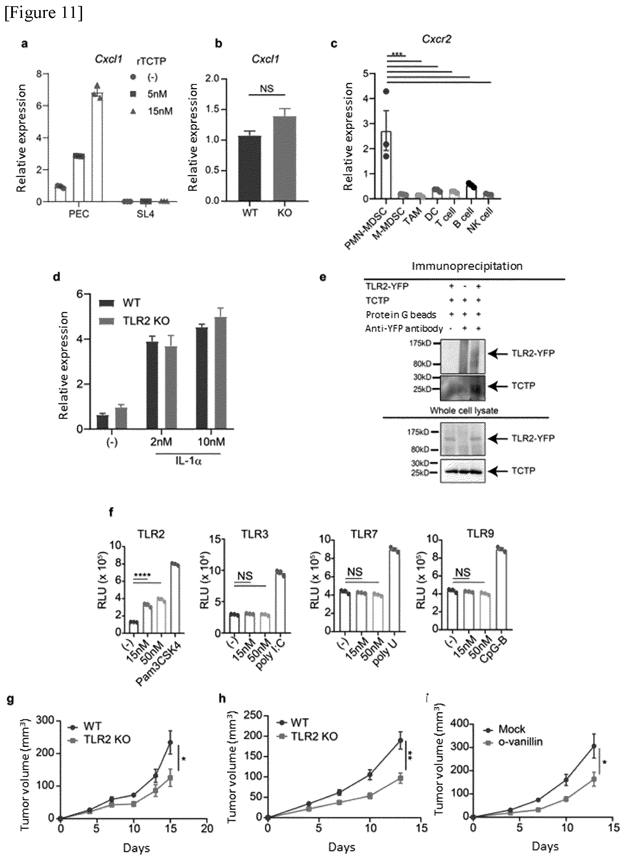

[Figure 11] Figure 11 shows identification of cells targeted by TCTP and TCTP receptor (2). In Figure 11(a), PECs (2 × 10⁵ cells) or SL4 cells (1 × 10⁵ cells) were stimulated with recombinant TCTP for 2 hours. Thereafter, Cxcl1 mRNA was quantified by qRT-PCR (n = 3). In Figure 11(b), the Cxcl1 mRNA of TCTP WO SL4 cells and TCTP KO SL4 cells was quantified by RT-qPCR. n = 3. In Figure 11(c), SL4 cells (2 × 10⁵ cells) were subcutaneously transplanted into C57BL/6 mice (n = 3). On Day 21 after the transplantation, single cell suspensions were prepared from tumors. Thereafter, various immune cells (PMN-MDSCs, M-MDSCs, TAMs, DCs, T cells, B cells, and NK cells) were separated, and the Cxcl2 mRNA of individual cells was then quantified by qRT-PCR. In Figure 11(d), PECs (2 × 10⁵ cells) were stimulated with recombinant IL-1α for 2 hours. Thereafter, Cxcl1 mRNA was quantified by qRT-PCR (n = 3). Figure 11(e) shows an immunoprecipitation assay regarding the binding of TCTP to TLR2. HEK293T cells transiently expressing TLR2-YFP and Flag-TCTP were subjected to immunoprecipitation with an anti-GFP antibody, and thereafter, were analyzed with an anti-Flag antibody (upper view), and TLR2 and TCTP in the whole cell lysate were detected (lower view). In Figure 11(f), a luciferase reporter construct containing multimerized NFκB binding motifs and an expression vector for various proteins were transfected into HEK293T cells. Twenty-four hours after the transfection, the HEK293T cells (2 × 10⁴ cells) were seeded in a 96-well plate, and were then stimulated with recombinant TCTP or an agonist for each TLR (Pam3CSK4: 300 ng/ml, poly I:C: 100 µg/ml, poly U: 10 µg/ml, and CpG-M: 1 µM). Six hours after the simulation, cell lysates were extracted and were then subjected to a luciferase assay. In Figures 11(g) and (h), SL4 cells (g) or IL-2ss-TCTP cells (h) (2 × 10⁵ cells) were subcutaneously transplanted into WT mice (SL4: n = 7, IL-2ss-TCTP: n = 6) or TLR2 KO mice (SL4: n = 5, IL-2ss-TCTP: n = 6). The tumor volume was measured over time. In Figure 11(i), IL-2ss-TCTP SL4 cells (2 × 10⁵ cells) were subcutaneously transplanted into C57BL/6 mice, and the tumor volume was then measured over time. After transplantation of the tumor, O-vanillin (50 mg/kg) was orally administered to the mice every three days. Regarding Figures 11(b), (f), and (g) to (i), *P < 0.05, **P < 0.01, ***P < 0.001, ****P < 0.0001, and unpaired two-sided Student's t-test. Regarding Figure 11(c), repeated measures one-way ANOVA with Dunnett's multiple comparisons test. NS indicates "no significant difference." The data are shown as a mean value ± standard deviation (s.e.m.).

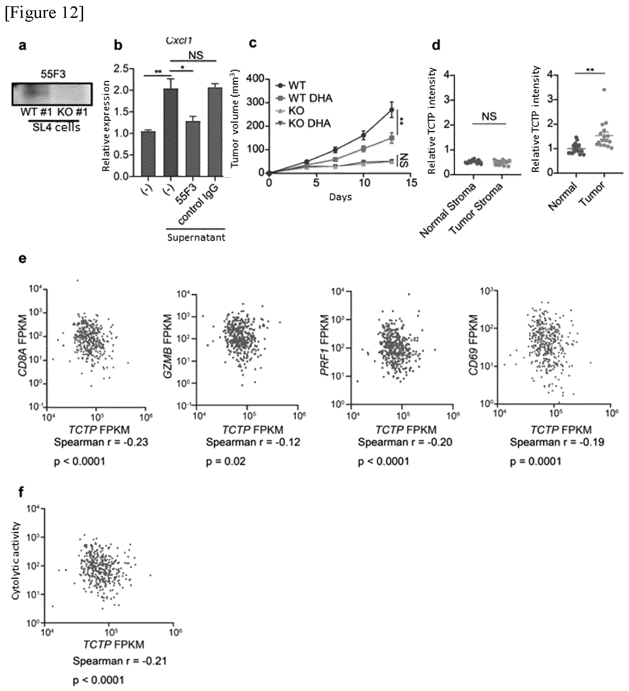

[Figure 12] Figure 12 shows studies regarding the influence of a TCTP neutralizing antibody and a TCTP inhibitor on tumor proliferation (1). Figure 12(a) shows the results obtained by preparing the whole cell lysate of TCTP WT SL4 cells (WT) or TCTP KO SL4 cells (KO), followed by immunoblotting using a 55F3E5 (55F3) monoclonal antibody. In Figure 12(b), PECs (2 × 10⁵ cells) were stimulated with a supernatant of dead SL4 cells comprising the 55F3 antibody or control IgG. After the stimulation for 2 hours, the expressed Cxcl1 mRNA was quantified by RT-qPCR. n = 3, and Sup: culture supernatant. In Figure 12(c), TCTP WT SL4 cells or TCTP KO SL4 cells (1 × 5¹⁰ cells) were subcutaneously transplanted into C57BL/6 mice. After the transplantation, DHA (50 mg/kg) or DMSO was intraperitoneally administered to the mice every day (n = 6). In Figure 12(d), (left view) the expression level of TCTP in the interstitial area of normal large intestinal tissues was compared with the expression level of TCTP in the interstitial area of colorectal cancer tissues. In Figure 12(d), (right view) a normal colon crypt was compared with a neoplastic lesion.
Figure 12(e) shows co-expression plots of CD8A, GZMB, PRF1 or CD69, and TCTP mRNA in a TCGA colorectal cancer dataset (n = 382). Figure 12(f) shows co-expression plots of cytolytic activity (defined as the geometric mean of the expression levels of GZMA mRNA and PRF1 mRNA) and TCTP mRNA in a TCGA colorectal cancer dataset (n = 382). Spearman r: spearman correlation coefficients. *P < 0.05, **P < 0.01. Regarding Figure 12(b), repeated measures one-way ANOVA with Dunnett's multiple comparisons test. Regarding Figure 12(c), repeated measures one-way ANOVA with Tukey's multiple comparisons test. Regarding Figure 12(d), unpaired two-sided Student's t-test. NS indicates "no significant difference." The data are shown as a mean value ± standard deviation (s.e.m.).

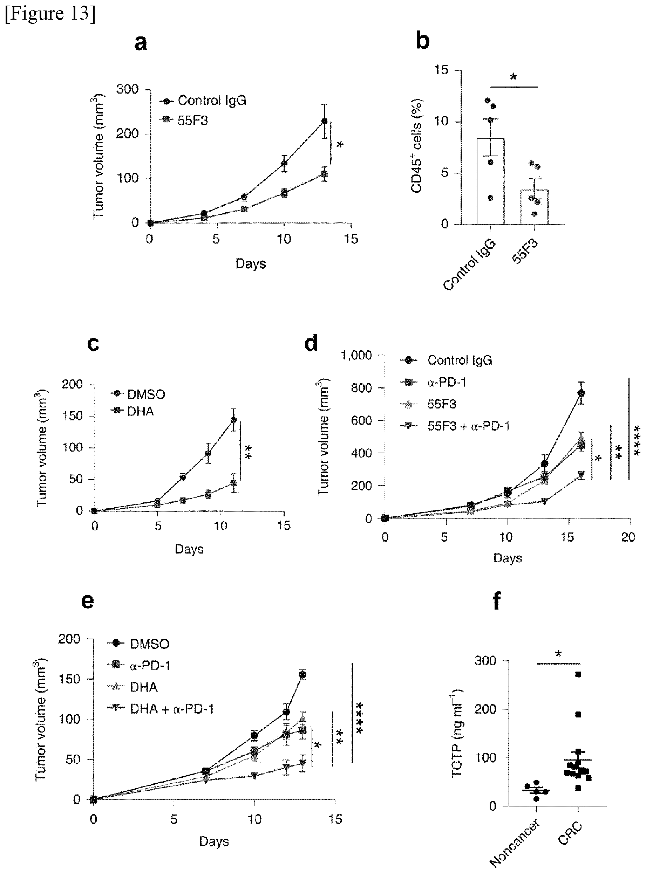

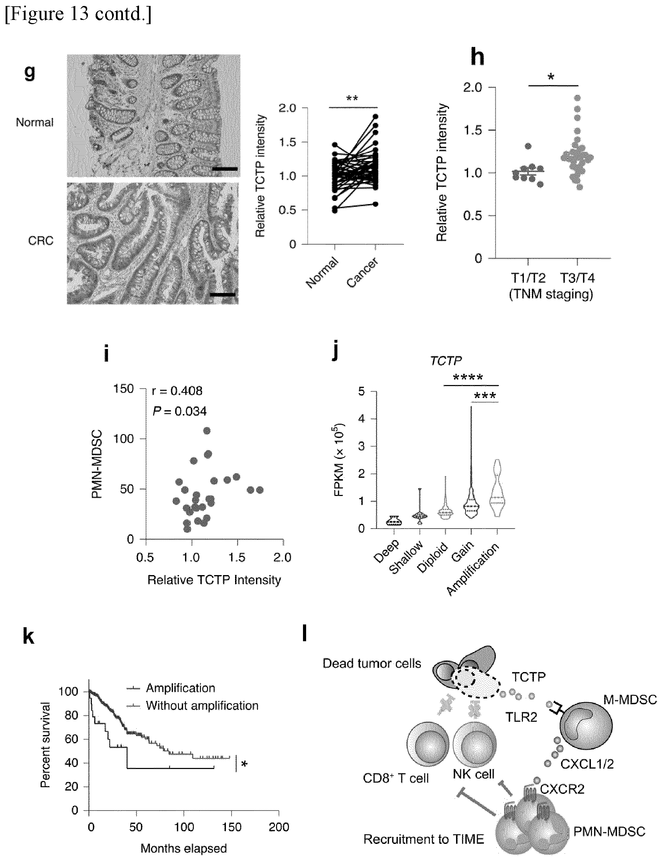

[Figure 13] Figure 13 shows studies regarding the influence of a TCTP neutralizing antibody and a TCTP inhibitor on tumor proliferation (2). In Figure 13(a), SL4 cells (1 × 10⁵ cells) were subcutaneously transplanted into C57BL/6 mice. The monoclonal antibody (55F3) (n = 5) reacting against TCTP or control IgG (n = 6) was intraperitoneally administered to the mice, every other day from one day after the transplantation (200 µg/mouse). The tumor volume was measured over time. In Figure 13(b), on Day 13 after the transplantation, single cell suspensions were prepared from tumors, and were then subjected to a flow cytometry analysis (n = 5). The longitudinal axis shows the abundance ratio of PMN-MDSCs to CD45⁺ cells. In Figure 13(c), SL4 cells (1 × 10⁵ cells) were subcutaneously transplanted into C57BL/6 mice (n = 6). After the transplantation, DHA (50 mg/kg) was intraperitoneally transplanted into the mice every day. The tumor volume was measured over time. In Figure 13(d), SL4 cells (1 × 10⁵ cells) were subcutaneously transplanted into C57BL/6 mice (n = 6). The 55F3 antibody or control IgG (200 µg/mouse, each) was intraperitoneally transplanted into the mice every day from one day after the transplantation. Ten days after the transplantation, an anti-PD-1 monoclonal antibody (100 µg) or PBS was administered to the mice. The tumor volume was measured over time. In Figure 13(e), SL4 cells (1 × 10⁵ cells) were subcutaneously transplanted into C57BL/6 mice. After the transplantation, DMSO, DHA, DHA together with an anti-PD-1 antibody (n = 5, each), or an anti-PD-1 antibody (n = 6), was administered to the mice. After the transplantation, DHA (50 mg/kg) or DMSO was intraperitoneally administered to the mice every day. On Day 6 after the transplantation, an anti-PD-1 antibody (100 µg) or PBS was administered to the mice. In Figure 13(f), the amount of TCTP in sera derived from healthy subjects (people who are not affected with cancer) (n = 5) and colorectal cancer patients (n = 14) was quantified by immunoblotting. In Figure 13(g), the left view shows representative examples of stained images of human colorectal cancer tissues (CRC) and normal large intestinal tissues (Normal) stained with hematoxylin and an anti-TCTP antibody. The scale indicates 100 µm. In Figure 13 (g), (right view) the relative staining intensity by the anti-TCTP antibody was semi-quantified, and the staining intensity of the colorectal cancer tissues was compared with the staining intensity of the normal large intestinal tissues (n = 45). In Figure 13(h), the expression level of TCTP in T1/2 colorectal cancer tissues (n = 9) was compared with the expression level of TCTP in T3/4 colorectal cancer tissues (n = 35). In Figure 13(i), human colorectal cancer tissues were stained with an anti-CD15 antibody and an anti-TCTP antibody. The number of CD15⁺ cells present in a region of 40,000 µm² in the visual fields was counted, and the relative staining intensity by the anti-TCTP antibody was then semi-quantified (n = 27). These are co-expression plots of CD15 and TCTP. It shows Spearman correlation coefficient. Figure 13(j) shows FPKM (Fragments Per Kilobasse of transcript per Million mapped reads) of TCTP stratified by the DNA copy number of CRC patients (n = 376) obtained from TCGA database. Deep (n = 4) or Shallow (n = 14) patients each delete 1 or 2 alleles of TCTP genes. Gain (n = 220) or Amplification (n = 16) patients each acquire 1 or more alleles of TCTP genes. Diploid, n = 122. Figure 13(k) is a Kaplan-Meier graph of TCGA data showing the survival of rectal cancer patients with TCTP allele amplification (n = 19) or rectal cancer patients without TCTP allele amplification (n = 520). Figure 13(l) is a general view of the present invention. Regarding Figures 13 (a) to (c), (f), and (h), *P < 0.05, **P < 0.01, ****P < 0.0001, and unpaired two-sided Student's t-test. Log-rank test (Figure 13(g)). Regarding Figures 13(d) and (e), repeated measures one-way ANOVA with Dunnett's multiple comparisons test. Regarding Figure 13(j), repeated measures one-way ANOVA with Tukey's multiple comparisons test. Regarding Figure 13(g), paired two-sided t-test. The data are shown as a mean value ± standard deviation (s.e.m.).

### Description of Embodiments

Hereinafter, the embodiments of the present invention will be described.

A first embodiment of the present invention relates to an inhibitor of the accumulation of myeloid-derived suppressor cells (MDSCs) in the tumor microenvironment (TIME), wherein the inhibitor comprises, an active ingredient, a substance that suppresses or inhibits the function of an immunomodulator released from dead tumor cells (hereinafter also referred to as "the inhibitor according to the present embodiment").

Herein, the tumor immune microenvironment (hereinafter also referred to as "TIME") means a site of interaction between cancer cells and non-cancer cells mainly including immune cells, and the tumor immune microenvironment is defined to be a microenvironment present in cancer, which comprises an environment advantageous for the survival of cancer, including advanced immunosuppression as a typical example (Non Patent Literature 1). Myeloid-derived suppressor cells (which are also referred to as "MDSCs") are immature myeloid cells appearing in infectious disease or chronic inflammation such as cancer, and having strong immunosuppressive activity. Monocytic myeloid-derived suppressor cells (which are also referred to as "M-MDSCs") and polymorphonuclear myeloid-derived suppressor cells (which are also referred to as "PMN-MDSCs") constitute principal subpopulations, and these two types of cells are similar to monocytes and neutrophils, respectively, in terms of forms and cell surface markers (Non Patent Literature 3). Accumulation of MDSCs in TIME means that MDSCs such as PMN-MDSCs are located in the TIME or around the TIME.

Herein, the immunomodulator means a factor that promotes or suppresses immune response and influences on the function of the immune system.

The present inventors have found that, when TCTP (a translationally controlled tumor protein) as a molecule released from dead tumor cells acts on blood cells present in the TIME, cytokines such as CXCL1 and CXCL2 are significantly released from, in particular, M-MDSCs, among those blood cells. Moreover, the present inventors have also found that CXCL1 and CXCL2 induce PMN-MDSCs into or around the tumor immune microenvironment, mediated by CXCR2 receptors present on the PMN-MDSCs. It has been known that the PMN-MDSCs induced into or around the tumor immune microenvironment suppress the attack of immune cells such as T cells or NK cells against cancer cells (Non Patent Literature 3). According to the present analysis, it has been newly clarified that the antitumor activity of immune cells is suppressed by accumulation of PMN-MDSCs in TIME through the aforementioned TCTP-(M-MDSC)-CXCL1/2-(PMN-MDSC) pathway, so that tumor proliferation can be promoted. Accordingly, if accumulation of MDSCs (for example, PMN-MDSCs) in the TIME were inhibited, accumulation or activation of T cells, NK cells, etc. could be promoted, and as a result, tumor proliferation could be inhibited or suppressed.

In order to inhibit accumulation of PMN-MDSCs in the TIME, for example, it is conceived to block the TCTP-(M-MDSC)-CXCL1/2-(PMN-MDSC) pathway. The method of blocking this pathway may be, for example, the use of a substance that suppresses or inhibits the function of TCTP (regarding human TCTP, NCBI Accession No.; cDNA sequence: CCDS9397.1, and amino acid sequence: NP_003286.1). Herein, the function of TCTP may be, for example, the function of TCTP to bind to a receptor thereof (e.g. TLR2) and to induce the release of cytokines (e.g. CXCL1/2) from cells (e.g. M-MDSCs, etc.).

Examples of the substance that suppresses or inhibits the function of TCTP may include, but are not particularly limited to: antibodies, peptide aptamers, and the like that suppress or inhibit the function of TCTP; substances that decompose TCTP or induce decomposition of TCTP, such as, for example, dihydroartemisinin (DHA) and Sertraline; and substances that suppress or inhibit the expression of TCTP, such as, for example, siRNA and miRNA. Further examples of the substance that suppresses or inhibits the function of TCTP may also include substances that inhibit the binding of TCTP to a receptor thereof (TLR2), such as, for example, a TLR2 antagonist. This inhibitory substance may be a substance that interacts with TCTP or a receptor thereof (TLR2), or decomposes any one of them.

The inhibitor according to the present embodiment may comprise the above-described substance that suppresses or inhibits the function of TCTP.

A second embodiment of the present invention relates to an antibody that suppresses or inhibits the function of TCTP (hereinafter also referred to as "the anti-TCTP antibody according to the present embodiment").

The "antibody" used in the present description is not particularly limited in terms of a preparation method thereof and a structure thereof, and examples of the present antibody may include all "antibodies" that each bind to a desired antigen based on desired properties, such as, for example, a monoclonal antibody, a polyclonal antibody, or a nanoantibody.

When the anti-TCTP antibody according to the present embodiment is a polyclonal antibody, the anti-TCTP antibody can be prepared, for example, by injecting a mixture of an antigen and an adjuvant into an animal to be immunized (which, for example, includes, but is not limited to, a rabbit, a goat, sheep, a chicken, a Guinea pig, a mouse, a rat, a pig, etc.). In general, an antigen and/or an adjuvant are injected into the subcutis or abdominal cavity of such an animal to be immunized several times. Examples of the adjuvant may include, but are not limited to, complete Freund and monophosphoryl lipid A synthesis-trehalose dicholinemicolate (MPL-TMD). After immunization with the antigen, the anti-TCTP antibody can be purified from the serum derived from the immunized animal by a conventional method (for example, a method using Sepharose that carries Protein A, etc.).

On the other hand, when the anti-TCTP antibody according to the present embodiment is a monoclonal antibody, the anti-TCTP antibody can be produced, for example, as follows. Besides, the term "monoclonal" is used in the present description to suggest the properties of an antibody obtained from a population of substantially uniform antibodies (i.e. an antibody population, in which the amino acid sequences of heavy chains and light chains constituting the antibodies are identical to one another), and thus, it should not be restrictively interpreted that the antibody is produced by a specific method (e.g. a hybridoma method, etc.).

Examples of the method of producing a monoclonal antibody may include a hybridoma method (Kohler and Milstein, Nature 256, 495-497, 1975) and a recombination method (U.S. Patent No. 4,816,567). Otherwise, the anti-TCTP antibody according to the present embodiment may be isolated from a phage antibody library (for example, Clackson et al., Nature 352, 624-628, 1991; Marks et al., J. Mol. Biol. 222, 581-597, 1991; etc.) and the like. More specifically, when a monoclonal antibody is prepared by applying a hybridoma method, the preparation method includes, for example, the following 4 steps: (i) immunizing an animal to be immunized with an antigen, (ii) recovering monoclonal antibody-secreting (or potentially secreting) lymphocytes, (iii) fusing the lymphocytes with immortalized cells, and (iv) selecting cells that secrete a desired monoclonal antibody. Examples of the animal to be immunized that can be selected herein may include a mouse, a rat, a Guinea pig, a hamster, and a rabbit. After completion of the immunization, in order to establish hybridoma cells, lymphocytes obtained from a host animal are fused with an immortalized cell line, using a fusion agent such as polyethylene glycol, or an electrical fusion method. As fusion cells, for example, a rat or mouse myeloma cell line is used. After completion of the cell fusion, the cells are allowed to grow in a suitable medium containing a substrate that inhibits the growth or survival of unfused lymphocytes and immortalized cell line. According to an ordinary technique, parent cells that lack the enzyme, hypoxanthine-guanine phosphoribosyl transferase (HGPRT or HPRT), are used. In this case, aminopterin is added to a medium that inhibits HGPRT-deficient cells and accepts the growth of hybridomas (i.e. HAT medium). From the thus obtained hybridomas, hybridomas generating desired antibodies are selected, and thereafter, a monoclonal antibody of interest can be obtained from a medium in which the selected hybridomas grow, according to an ordinary method.

The thus prepared hybridomas are cultured *in vitro,* or are cultured *in vivo* in the ascites fluid of a mouse, a rat, a Guinea pig, a hamster, etc., and an antibody of interest can be then prepared from the culture supernatant, or from the ascites fluid.

Nanoantibody is a polypeptide consisting of a variable region of an antibody heavy chain (i.e. a variable domain of the heavy chain of heavy chain antibody (VHH)). In general, an antibody of a human or the like is composed of heavy and light chains. However, animals of family Camelidae, such as llamas, alpacas and camels, produce single-chain antibodies (heavy chain antibodies) consisting only of heavy chains. Such a heavy chain antibody can recognize a target antigen and can bind thereto, as in the case of a common antibody consisting of heavy and light chains. The variable region of a heavy chain antibody is the smallest unit that has a binding affinity to an antigen, and this variable region fragment is called a "nanoantibody." Nanoantibodies have high heat resistance, digestion resistance, and room temperature stability, and can be easily prepared in large quantities by a genetic engineering technique.

A nanoantibody can be produced, for example, as follows. An animal of family Camelidae is immunized with an antigen, and the presence or absence of an antibody of interest is then detected from the collected serum. Thereafter, cDNA is prepared from RNA derived from the peripheral blood lymphocytes of an immunized animal, in which a desired antibody titer is detected. A DNA fragment encoding VHH is amplified from the obtained cDNA, and the amplified DNA fragment is then inserted into a phagemid to prepare a VHH phagemid library. A desired nanoantibody can be prepared from the prepared VHH phagemid library through several screenings.

The anti-TCTP antibody according to the present embodiment may be a genetically engineered antibody. Such a genetically engineered antibody is not limited, and examples thereof may include a humanized antibody, and a chimeric antibody with a human antibody. The chimeric antibody is, for example, an antibody, in which a variable region derived from a different animal species is linked with a constant region derived from another different animal species (for example, an antibody, in which a variable region of a rat-derived antibody is bound to a constant region derived from a human) (for example, Morrison et al., Proc. Natl. Acad. Sci. U.S.A. 81, 6851-6855, 1984., etc.). The chimeric antibody can be easily constructed by genetic recombination technology.

The humanized antibody is an antibody that has a human-derived sequence in the framework region (FR) thereof and has a complementarity determining region (CDR) consisting of a sequence derived from another animal species (for example, a mouse, etc.). When such a humanized antibody is first explained using another animal species, for example, using a mouse, CDRs are transplanted from the variable regions of a mouse-derived antibody into human antibody variable regions, so that the heavy chain and light chain variable regions of the human antibody are reconstituted. Thereafter, the humanized reconstituted human antibody variable regions are ligated to humanized antibody constant regions, so that a humanized antibody can be produced. The method for producing such a humanized antibody is publicly known in the present technical field (e.g. Queen et al., Proc. Natl. Acad. Sci. USA, 86, 10029-10033, 1989., etc.).

The antigen-binding fragment of the anti-TCTP antibody according to the present embodiment is a partial region of the anti-TCTP antibody according to the present embodiment, which is an antibody fragment that binds to TCTP. Examples of such an antigen-binding fragment may include Fab, Fab', F(ab')₂, Fv (a variable fragment of an antibody), a single chain antibody (a heavy chain, a light chain, a heavy chain variable region, a light chain variable region, a nanoantibody, etc.), scFv (single chain Fv), a diabody (an scFv dimer), dsFv (disulfide-stabilized Fv), and a peptide comprising the CDR of the anti-TCTP antibody according to the present embodiment, at least, as a part thereof.

Fab is an antibody fragment having antigen-binding activity, in which about a half of the N-terminal side of a heavy chain and a light chain as a whole are bound to each other via a disulfide bond, among fragments obtained by treating an antibody molecule with the proteolytic enzyme papain. Such Fab can be produced by treating an antibody molecule with papain to obtain a fragment, and also, for example, by constructing a suitable expression vector into which DNA encoding Fab is inserted, then introducing this vector into suitable host cells (e.g. mammalian cells such as CHO cells, yeast cells, insect cells, etc.), and then allowing Fab to express in the cells.

F(ab')₂ is an antibody fragment having antigen-binding activity, which is slightly larger than a fragment obtained by treating an antibody molecule with the proteolytic enzyme pepsin, in which Fab is bound via a disulfide bond in the hinge region. Such F(ab')₂ can be produced by treating an antibody molecule with pepsin to obtain a fragment, or via a thioether bond or a disulfide bond, or further, by a genetic engineering technique, as in the case of Fab.

Fab' is an antibody fragment having antigen-binding activity, in which the disulfide bond in the hinge region of the above-described F(ab')₂ is cleaved. Such Fab' can also be produced by a genetic engineering technique, as in the case of Fab.

scFv is a VH-linker-VL or VL-linker-VH polypeptide, in which one heavy chain variable region (VH) and one light chain variable region (VL) are linked to each other using a suitable peptide linker, and it is an antibody fragment having antigen-binding activity. Such ScFv can be produced by obtaining cDNAs encoding the heavy and light chain variable regions of an antibody, and then performing a genetic engineering technique.

Diabody is an antibody fragment having a divalent antigen-binding activity, in which scFv is dimerized. The divalent antigen-binding activity may be an identical antigen-binding activity, or one of them may be a different antigen-binding activity. Such a diabody can be produced by obtaining cDNAs encoding the heavy chain and light chain variable regions of an antibody, then constructing cDNA encoding scFv, in which the heavy chain variable region and the light chain variable region are linked to each other by a peptide linker, and then performing a genetic engineering technique.

DsFv refers to polypeptides, in which one amino acid residue in each of the heavy chain variable region and the light chain variable region is replaced with a cysteine residue, which are bound to each other via a disulfide bond between the cysteine residues. The amino acid residue to be replaced with the cysteine residue can be selected based on the prediction of the three-dimensional structure of the antibody. Such dsFv can be produced by obtaining cDNAs encoding the heavy chain and light chain variable regions of an antibody, then constructing DNA encoding the dsFv, and then performing a genetic engineering technique.

A peptide comprising a CDR is configured to comprise at least one region of the CDRs (CDR1 to 3) of a heavy or a light chain. A plurality of peptides each comprising a CDR can be bound to one another, directly or via a suitable peptide linker. Such a peptide comprising a CDR can be produced by constructing DNA encoding the CDR of the heavy chain or light chain of an antibody, and inserting the constructed DNA into an expression vector. Herein, the type of the vector is not particularly limited, and it may be appropriately selected, depending on the types of host cells into which the vector is to be introduced, etc. Thereafter, the peptide comprising a CDR can be produced by introducing the expression vector comprising the DNA into suitable host cells (e.g. mammalian cells such as CHO cells, yeast cells, insect cells, etc.) for allowing it to express as an antibody. Alternatively, the peptide comprising a CDR can also be produced by a chemical synthesis method such as an Fmoc method (fluorenylmethyloxycarbonyl method) and a tBoc method (t-butyloxycarbonyl method).

In general, in the case of a human antibody (a complete human antibody), a hypervariable region that is the antigen binding site of a V region, other parts of the V region, and a constant region have the same structures as those of the antibody of a human. Such a human antibody can be easily produced by a person skilled in the art according to a known technique. The human antibody can be obtained by, for example, a method using a human antibody-producing mouse having a human chromosome fragment containing the H chain and L chain genes of the human antibody (e.g. Tomizuka et al., Proc. Natl. Acad. Sci. USA, 97, 722-727, 2000., etc.) or a method of obtaining a human antibody derived from a phage display selected from a human antibody library (see, for example, Siriwardena et al., Opthalmology, (2002) 109 (3), 427-431, etc.).

A multispecific antibody can be constructed using the antigen-binding fragment of the anti-TCTP antibody according to the present embodiment. Multispecificity means that an antibody has binding specificity to two or more antigens, and may be, for example, the form of a protein containing a monoclonal antibody having binding specificity to two or more antigens or an antigen-binding fragment thereof. Such multi specificity is achieved by a person skilled in the art according to a known technique. As methods of constructing multi specificity, there have been developed multiple methods, which are classified into a technique of constructing an asymmetric IgG, in which two different types of antibody heavy chain molecules are subjected to protein engineering operations so that they form a heterodimer, and a technique of ligating to each other, antigen-binding fragments each having a low molecular weight, which are obtained from an antibody, or ligating such an antigen-binding fragment to another antibody molecule. As an example of a specific construction method, the following publication can be, for example, referred to: Kontermann et al., Drug Discovery Today, 20, 838-847, 2015.

Examples of the anti-TCTP antibody according to the present embodiment and an antigen-binding fragment thereof may include antibodies, which are characterized in that the amino acid sequences of CDRs (complementarity determining regions) 1 to 3 satisfy any of the following (A), (B) or (C), and antigen-binding fragments thereof.
(A) CDRs of 55F3 antibody have:
   the amino acid sequence of heavy chain CDR1 that is GYSIASDYAWN (SEQ ID NO: 1),
   the amino acid sequence of heavy chain CDR2 that is YINYSGSTGYNPSLKS (SEQ ID NO: 2),
   the amino acid sequence of heavy chain CDR3 that is FEAGY (SEQ ID NO: 3),
   the amino acid sequence of light chain CDR1 that is KASQDINRYLS (SEQ ID NO: 4),
   the amino acid sequence of light chain CDR2 that is RANRLVD (SEQ ID NO: 5), and
   the amino acid sequence of light chain CDR3 that is LQYNEFPLT (SEQ ID NO: 6).
(B) CDRs of 44E1 antibody have:
   the amino acid sequence of heavy chain CDR1 that is GYTFTDHAIH (SEQ ID NO: 7),
   the amino acid sequence of heavy chain CDR2 that is YISPGNGDLKYNEKFKG (SEQ ID NO: 8),
   the amino acid sequence of heavy chain CDR3 that is GWTL (SEQ ID NO: 9),
   the amino acid sequence of light chain CDR1 that is KSSQSLLYRSNQKNYLV (SEQ ID NO: 10),
   the amino acid sequence of light chain CDR2 that is WAFTRES (SEQ ID NO: 11), and
   the amino acid sequence of light chain CDR3 that is QQHYSYPWT (SEQ ID NO: 12).
(C) CDRs of 51A9 antibody have:
   the amino acid sequence of heavy chain CDR1 that is GYSITSDYAWN (SEQ ID NO: 13),
   the amino acid sequence of heavy chain CDR2 that is YINYSGSTGYNPSLKS (SEQ ID NO: 14),
   the amino acid sequence of heavy chain CDR3 that is FEAGY (SEQ ID NO: 15),
   the amino acid sequence of light chain CDR1 that is KASQDINSYLS (SEQ ID NO: 16),
   the amino acid sequence of light chain CDR2 that is RANRLVD (SEQ ID NO: 17), and
   the amino acid sequence of light chain CDR3 that is LQYYEFPLT (SEQ ID NO: 18).

Moreover, the anti-TCTP antibody according to the present embodiment and an antigen-binding fragment include: an antibody comprising any of heavy chain variable regions comprising the amino acid sequence as set forth in SEQ ID NO: 19, SEQ ID NO: 20, or SEQ ID NO: 21, an antibody comprising any of light chain variable regions comprising the amino acid sequence as set forth in SEQ ID NO: 22, SEQ ID NO: 23, or SEQ ID NO: 24, and antigen-binding fragments thereof; and an antibody consisting of an amino acid sequence having an amino acid sequence identity of about 70% or more, preferably, about 80% or more, about 81% or more, about 82% or more, about 83% or more, about 84% or more, about 85% or more, about 86% or more, about 87% or more, about 88% or more, or about 89% or more, more preferably, about 90% or more, about 91% or more, about 92% or more, about 93% or more, about 94% or more, about 95% or more, about 96% or more, about 97% or more, or about 98% or more, and most preferably about 99% or more, to the amino acid sequence of each of a heavy chain variable region and/or a light chain variable region that constitute the aforementioned antibodies, wherein the antibody inhibits the binding of TCTP to a receptor thereof, and an antigen-binding fragment thereof.

### Heavy chain variable region of 55F3 antibody

MRVLILLWLFTAFPGILSDVQLQESGPGLVKPSQSLSLTCTATGYSIASDYAWNW
IRQFPGNKLEWMGYINYSGSTGYNPSLKSRISITRDTSKNQFFLQLNSVTTEDTA
TYYCARFEAGYWGQGTTLTVSSAKTTPPSVYPLAPGSAAQTNSMVTLGCLVK
GYFPEPVT (SEQ ID NO: 19)

### Heavy chain variable region of 44E1 antibody

MEWSWVFLFFLSVTTGVHSEVQLQQSDAELVKPGASVKISCKASGYTFTDHAI

HWAKQKPEQGLEWIGYISPGNGDLKYNEKFKGKATLTTDKSSSTAYMQLNSLT
SEDSAVYFCKSGWTLWGQGTTLTVSSAKTTPPSVYPLAPGSAAQTNSMVTLGC
LVKGYFPEPVT (SEQ ID NO: 20)

### Heavy chain variable region of51A9 antibody

MRVLILLWLFTAFPGILSDVQLQESGPGLVKPSQSLSLTCTATGYSITSDYAWNW
IRQFPGNKLEWMGYINYSGSTGYNPSLKSRISITRDTSKNKFFLQLNSVTTEDTA
TYYCARFEAGYWGQGTTLTVSSAKTTPPSVYPLAPGSAAQTNSMVTLGCLVK
GYFPEPVT (SEQ ID NO: 21)

### Light chain variable region of 55F3 antibody

MDMRTPAQFLGILLLWFPGIKCDIKMTQSPSSMSASLGERVTITCKASQDINRYL
SWFQQKPGKSPKTLIYRANRLVDGVPSRFSGSGSGQDYSLTISSLEYGDMGIYY
CLQYNEFPLTFGAGTKLELKRADAAPTVSIFPPSSEQLTSGGASVVCFLN (SEQ
ID NO: 22)

### Light chain variable region of 44E1 antibody

MDSQAQVLMLLLLWVSGTCGDIVMSQSPSSPVVSVGEKVTMSCKSSQSLLYRS
NQKNYLVWYQLKPGQSPKLLIYWAFTRESGVPDRFTGSGSGTDFTLTISSVKAE
DLAVYYCQQHYSYPWTFGGGTKLEVKRADAAPTVSIFPPSSEQLTSGGASVVC
FLN (SEQ ID NO: 23)

### Light chain variable region of51A9 antibody

MDMRTPAQFLGILLLWFPGIKCDIKMTQSPSSMYASLGERVTFTCKASQDINSY
LSWFQQKPGKSPKTLIYRANRLVDGVPSRFSGSGSGQDYSLTISSLDYEDMGIY
YCLQYYEFPLTFGAGTKLELKRADAAPTVSIFPPSSEQLTSGGASVVCFLN (SEQ
ID NO: 24).

Furthermore, the anti-TCTP antibody according to the present embodiment also includes an antibody that competitively inhibits the binding of the antibody characterized in that the amino acid sequences of CDRs (complementarity determining regions) 1 to 3 satisfy any of the above-described (A), (B) or (C), to TCTP, and that suppresses or inhibits the function of TCTP (hereinafter also referred to as "the competitive antibody according to the present embodiment"). The competitive antibody according to the present embodiment can be prepared and obtained by a competitive experiment and the like that are publicly known to a person skilled in the art. Specifically, when the binding of a first anti-TCTP antibody (the anti-TCTP antibody according to the present embodiment) to TCTP is competitively inhibited by a second anti-TCTP antibody, it is judged that the first anti-TCTP antibody and the second anti-TCTP antibody bind to a substantially identical antigen site, or that they bind to antigen sites that are extremely close to each other. When the second anti-TCTP antibody suppresses or inhibits the function of TCTP, the second anti-TCTP antibody is the competitive antibody according to the present embodiment, and is included in the anti-TCTP antibody according to the present embodiment. Besides, as a method of the above-described competitive experiment, for example, a method of using a Fab fragment or the like is generally carried out in the present technical field. Please refer to, for example, WO95/11317, WO94/07922, WO2003/064473, WO2008/118356, WO2004/046733, etc. Moreover, whether or not the second anti-TCTP antibody suppresses or inhibits the function of TCTP can be easily confirmed by the method disclosed in the after-mentioned Examples.

Further, the anti-TCTP antibody according to the present embodiment also includes an antibody that binds to a partial peptide of TCTP that is EDGVTPYMIFFKDGLEMEKC (SEQ ID NO: 25) and suppresses or inhibits the function of TCTP.

A third embodiment of the present invention relates to a therapeutic drug or composition for cancer, comprising the inhibitor of the present invention as an active ingredient (hereinafter referred to as a "therapeutic drug or the like") (the therapeutic drug or the like according to the embodiment of the present invention).

As a therapeutic drug for cancer according to the embodiment of the present invention, the active ingredient itself (e.g. a substance that suppresses or inhibits the function of TCTP, etc.) may be administered. However, in general, the therapeutic drug for cancer according to the embodiment of the present invention is desirably administered in the form of a therapeutic composition comprising one or two or more pharmaceutical additives, as well as one or more substances serving as an active ingredient(s). The therapeutic drug or the like of the present invention may comprise, as active ingredients, a plurality of different inhibitors of the present invention. Moreover, the therapeutic drug or the like of the present invention may also comprise known components of other anticancer agents, immune checkpoint inhibitors, and the like.

The dosage forms of the therapeutic drug or the like according to the embodiment of the present invention may include tablets, capsules, granules, powder agents, syrups, suspensions, suppositories, ointments, creams, gelling agents, patches, inhalants, and injections. These preparations are produced according to ordinary methods. Liquid preparation may be dissolved or suspended in water or another suitable solvent at the time of use. In addition, tablets and granules may be coated by a publicly known method. Injections are prepared by dissolving the active ingredient in water. As necessary, the active ingredient of the injection may be dissolved in a normal saline or a glucose solution, and further, a buffer agent or a preservative may be added to such a solution.

A preparation for use in oral administration or parenteral administration is provided in any given preparation form. Examples of the preparation form that can be prepared herein may include: therapeutic drugs or compositions for use in oral administrations, having forms such as granules, fine granules, powder agents, hard capsules, soft capsules, syrups, emulsions, suspensions, or liquid agents; and therapeutic drugs or compositions for use in parenteral administrations such as intravenous administration, intermuscular administration, or subcutaneous administration, having forms such as injections, drops, transdermal absorbents, transmucosal absorbents, nasal drops, inhalants, or suppositories. Such injections or drops can be prepared as powdery dosage forms such as freeze-dried forms, and can be then used by being dissolved in an appropriate aqueous medium such as a normal saline at the time of use.

The types of pharmaceutical additives used in production of the therapeutic drug or the like according to the embodiment of the present invention, the ratio of the pharmaceutical additives to the active ingredient, or a method for producing a pharmaceutical drug or a pharmaceutical composition can be appropriately selected by a person skilled in the art, depending on the forms thereof. As such pharmaceutical additives, inorganic or organic substances, or solid or liquid substances can be used. In general, such pharmaceutical additives can be mixed in an amount from 1% by weight to 90% by weight, based on the weight of the active ingredient. Specific examples of the pharmaceutical additives may include lactose, glucose, mannit, dextrin, cyclodextrin, starch, sucrose, magnesium aluminometasilicate, synthetic aluminum silicate, sodium carboxymethyl cellulose, hydroxypropyl starch, calcium carboxymethyl cellulose, ion exchange resin, methyl cellulose, gelatin, gum Arabic, hydroxypropyl cellulose, hydroxypropyl methyl cellulose, polyvinyl pyrrolidone, polyvinyl alcohol, light anhydrous silicic acid, magnesium stearate, talc, tragacanth, bentonite, veegum, titanium oxide, sorbitan fatty acid ester, sodium lauryl sulfate, glycerin, fatty acid glycerin ester, purified lanolin, glycerogelatin, polysorbate, macrogol, vegetable oil, wax, liquid paraffin, white petrolatum, fluorocarbon, nonionic surfactant, propylene glycol, and water.

In order to produce a solid preparation for use in oral administration, an active ingredient is mixed with excipient components, such as, for example, lactose, starch, crystalline cellulose, calcium lactate, or anhydrous silicic acid, to form a powder agent. Otherwise, as necessary, a binder such as white sugar, hydroxypropyl cellulose or polyvinyl pyrrolidone, a disintegrator such as carboxymethyl cellulose or calcium carboxymethyl cellulose, and the like are further added thereto, and the obtained mixture is then subjected to wet or dry granulation to form a granule. In order to produce a tablet, such a powder agent or a granule is directly used, or a lubricant such as magnesium stearate or talc is added thereto, and they are then subjected to tableting. Such a granules or a tablet can be coated with an enteric coating base material such as hydroxypropylmethyl cellulose phthalate or a methacrylic acid-methyl methacrylate polymer to form an enteric coated preparation. Otherwise, such a granule or tablet can be coated with ethyl cellulose, carnauba wax, or hydrogenated oil to form a prolonged action preparation. Moreover, in order to produce a capsule, a powder agent or a granule is filled into a hard capsule. Otherwise, an active ingredient is directly used, or is dissolved in glycerin, polyethylene glycol, sesame oil, olive oil, etc., and the obtained mixture is then coated with a gelatin film, so that a soft capsule can be prepared.

In order to produce an injection, an active ingredient is dissolved in distilled water for injection, together with, as necessary, a pH adjuster such as hydrochloric acid, sodium hydroxide, lactose, lactic acid, sodium, sodium monohydrogen phosphate or sodium dihydrogen phosphate, and a tonicity agent such as sodium chloride or glucose, and thereafter, the obtained solution is subjected to aseptic filtration, and is then filled into an ampoule. Otherwise, mannitol, dextrin, cyclodextrin, gelatin, etc. are further added to the obtained solution, and the thus mixed solution is then subjected to vacuum-freeze drying, so that the injection may be prepared as an injection that is dissolved at the time of use. Alternatively, lecithin, polysorbate 80, polyoxyethylene hydrogenated castor oil, etc. can be added to the active ingredient, and they can be then emulsified in water to prepare an emulsion for injection.

In order to produce an agent for rectal administration, an active ingredient may be humidified and dissolved, together with a suppository base material such as cacao butter, fatty acid tri-, di- and mono-glyceride, or polyethylene glycol, and thereafter, the obtained mixture may be poured into a mold and may be then cooled. Otherwise, an active ingredient may be dissolved in polyethylene glycol, soybean oil or the like, and the obtained mixture may be then coated with a gelatin film.

The applied dose and the number of doses of the therapeutic drug or the like according to the embodiment of the present invention are not particularly limited, and the applied dose and the number of doses can be selected, as appropriate, by a doctor's judgement, depending on conditions such as the purpose of prevention and/or treatment of deterioration/progression of a therapeutic target disease, the type of the disease, and the body weight, age, etc. of a patient.

In general, the daily dose of the present therapeutic drug or the like for an adult by oral administration is approximately 0.01 to 1000 mg (the weight of the active ingredient), and the present therapeutic drug or the like can be administered once per day, or divided over several administrations per day. When the present therapeutic drug or the like is used in the form of an injection, it is desired to continuously or intermittently administer the therapeutic agent or the like to an adult at a daily dose of 0.001 to 100 mg (the weight of the active ingredient).

The therapeutic drug or the like according to the embodiment of the present invention may also be prepared in the form of a sustained release preparation, such as an implant and a delivery system encapsulated into a microcapsule, by using a carrier capable of preventing the immediate removal of the drug from the body. Examples of such a carrier that can be used herein may include biodegradable and biocompatible polymers such as ethylene vinyl acetate, polyacid anhydride, polyglycolic acid, collagen, polyorthoester, and polylactic acid. Such materials can be easily prepared by a person skilled in the art. In addition, a liposome suspension can also be used as a pharmaceutically acceptable carrier. Such a liposome is not limited, but can be prepared as a lipid composition comprising phosphatidylcholine, cholesterol and PEG-induced phosphatidyl ethanol (PEG-PE), by being passed through a filter having a suitable pore size, so that it can have a size suitable for the use thereof, and thereafter, the lipid composition can be purified by a reversed phase evaporation method.

The therapeutic drug or the like according to the embodiment of the present invention may be provided in the form of a kit together with an instruction manual regarding an administration method and the like. The drug included in the kit is supplied with a container produced with a material that effectively sustains the activity of structural components of the therapeutic drug or the like for a long period of time, is not adsorbed on the inner side of the container, and does not degenerate the structural components. For instance, a sealed glass ampoule may include a buffer or the like, which is enclosed in the presence of neutral and non-reactive gas such as nitrogen gas.

Moreover, the kit may be included with an instruction manual. The instruction manual of the kit may be printed out on a paper or the like, or may be stored in an electromagnetically readable medium such as CD-ROM or DVD-ROM and may be then supplied to users.

A third embodiment of the present invention relates to a method for preventing or treating cancer, comprising administering the therapeutic drug or the like according to the embodiment of the present invention (i.e. the second embodiment of the present invention) to a patient.

The term "to treat" means herein to stop or alleviate progression and deterioration of the pathological condition induced in a mammal affected with cancer. On the other hand, the term "to prevent" means herein to previously stop the onset of cancer in a mammalian patient which is likely to be affected with cancer. The "mammal" as a preventive or therapeutic target means any given animal classified into Mammals. Examples of the mammal may include, but are not particularly limited to, humans, companion animals such as dogs, cats, rabbits and ferrets, and living stock animals such as bovines, pigs, sheep and horses. A particularly preferred "mammal" is a human.

Examples of the cancer (malignant tumor/malignant neoplasm) as a preventive or therapeutic target of the preventive or therapeutic method of the present invention may include hepatocellular carcinoma, bile duct cell carcinoma, renal cell carcinoma, squamous cell carcinoma, basal cell carcinoma, transitional cell carcinoma, adenocarcinoma, malignant gastrinoma, malignant melanoma, fibrosarcoma, myxosarcoma, liposarcoma, leiomyosarcoma, rhabdomyosarcoma, malignant teratoma, angiosarcoma, Kaposi's sarcoma, osteosarcoma, chondrosarcoma, lymphangiosarcoma, malignant meningioma, non-Hodgkin lymphoma, Hodgkin lymphoma, leukemia, brain tumor, epithelial cell-derived neoplasm (epithelial carcinoma), basal cell carcinomas, adenocarcinomas, lip cancer, oral cancer, esophageal cancer, gastrointestinal cancer such as small bowel cancer and stomach cancer, colon cancer, rectal cancer, hepatic cancer, bladder cancer, pancreatic cancer, ovarian cancer, cervical cancer, lung cancer, breast cancer, skin cancers such as squamous cell carcinoma and basal cell carcinoma, prostate cancer, and renal cell carcinoma. In addition, examples of the cancer may also include known other cancers that affect the epithelium, mesenchyme, and blood cells in the whole body.

A fourth embodiment of the present invention relates to a method for diagnosing or auxiliarily diagnosing cancer, comprising measuring the amount of TCTP mRNA or TCTP protein that is present in a sample derived from a subject.

In a subject in whom cancer is developed, the amount of TCTP protein or TCTP mRNA in tumor tissues or in blood is significantly increased, in comparison to that in s healthy subject (a person in whom the onset of cancer is not confirmed) (see, for example, Figure 2(b), etc.). Accordingly, when the amount of TCTP mRNA or the amount of TCTP protein in a sample derived from a certain subject is significantly higher than that in a sample derived from a healthy subject, it can be determined that the certain subject is likely to be affected with some cancer.

In the present embodiment, the sample derived from a subject is not limited, but for example, blood (including components derived from blood, such as serum), tissues suspected to be a malignant tumor, or the like can be used. As a method of measuring the amount of TCTP protein in a sample, a person skilled in the art can easily select an appropriate method, and for example, an immunoassay, such as an ELISA (enzyme-linked immuno-sorbent assay) method or a Western blotting method, can be applied. In addition, as a method of measuring the amount of TCTP mRMA in a sample, a person skilled in the art can easily select an appropriate method, and for example, a real-time PCR (RT-qPCR) method can be applied.

When the present description is translated in English and the translation document includes singular terms with the articles "a," "an," and "the,", these terms include not only single items but also multiple items, unless otherwise clearly specified.

Hereinafter, the present invention will be further described in the following examples. However, these examples are only illustrative examples of the embodiments of the present invention, and thus, are not intended to limit the scope of the present invention.

### Examples

### 1. Methods

### 1-1. Mice

C57BL/6 and BALB/c mice were purchased from CLEA Japan Inc. Apc^{Δ716} mice were produced according to the previous report (19), and were used in the background of C57BL/6 mice. TCTP flox mice were kindly provided by Dr. Hsin-Fang Yang-Yen of Academia Sinica (Republic of China) and were used in the background of C57BL/6 mice. Villin-CreERT2 mice were kindly provided by Dr. Sylvie Robine of Institut Curie (France) and were used in the background of C57BL/6 mice. MyD88 and IPS-I-deficient mice were kindly provided by Dr. Shizuo Akira of Osaka University, and were used in the background of C57BL/6 mice. TLR2 and TLR4-deficient mice were purchased from Oriental Bio Service, Inc., and were used in the background of C57BL/6 mice. STING-deficient mice were kindly provided by Dr. Glen N. Barber of University of Miami (U.S.A.), and were used in the background of C57BL/6 mice. RAG1 knockout mice were kindly provided by Dr. Shinsuke Taki of Shinshu University. Both male and female mice (6-12 weeks old) have been used in the present Examples, unless otherwise specified. All animal experiments were approved by the University of Tokyo's Animal Research Committee.

### 1-2. Cells

RAW264.7 cells, the mouse melanoma cell line B16F10 cells, the mouse fibrosarcoma cell line Meth-A cells, and HEK293T cells were obtained from RIKEN BioResource Center (Japan). The mouse colon carcinoma cell line SL4 was kindly provided by Dr. T. Irimura (Juntendo University). RAW264.7 cells, B16F10 cells, and HEK293T cells were maintained in DMEM (Nacalai Tesque, Inc.) supplemented with 10% FBS (HyClone). Meth-A cells were maintained in RPMI (Nacalai Tesque, Inc.) supplemented with 10% FBS. SL4 cells were maintained in DMEM-F12 (Gibco) supplemented with 10% FBS. For preparation of peritoneal exudate cells (PECs), mice were intraperitoneally injected with 2 ml of 4% thioglycollate (DIFCO solution), and 4 days later, the abdominal cavity was washed with PBS to collect PECs. The cells were incubated in RPMI supplemented with 10% FBS on a petri dish overnight. After completion of the incubation, the cells were washed with an RPMI medium, and were then used in the subsequent experiments.

### 1-3. Flow cytometry analysis and fluorescence-activated cell sorting of tumor-infiltrating cells

Tumor-infiltrating cells were recovered after subcutaneous transplantation of a tumor into mice, and were then analyzed by flow cytometry. The excised tumor was finely minced, and was then treated with collagenase (0.75 mg/ml, cat# 11088882001, Roche), DNase I (40 µg/ml, cat# 11284932001, Roche), and dispase (0.5 mg/ml, cat# 17105041, Thermo Fisher Scientific), and thereafter, the resultant was intensively stirred (180 rpm, 37°C, 1 hour). The obtained cell suspension was passed through a cell strainer (cat# 352340, Falcon) and was then treated with an RBC lysis buffer (cat# 00-4333-57, Invitrogen). After washing with PBS, the cells were first incubated with an anti-CD16/32 antibody (clone 93, cat# 101302, BioLegend) on ice for 5 minutes. Thereafter, the cells were stained in PFE (PBS supplemented with 2 % FBS and 1 mM EDTA) on ice for 20 minutes. The resulting cells were analyzed by LSR Fortessa (BD Biosciences). Cell sorting of the tumor-infiltrating cells was performed using FACSAria II (BD Biosciences). The obtained data were analyzed with FlowJo software (BD BioSciences).

The following antibodies were used in flow cytometry analysis or fluorescence-activated cell sorting: Ly6C-AF488 (clone HK1.4, cat# 128022, BioLegend), NK1.1-FITC (clone PK136, cat# 553164, PharMingen), CD11b-PE (clone M1/70, cat# 101208, BioLegend), CD3ε-PE (clone 145-2C11, cat#12-0031-81, eBioscience), CD11c-APC (clone N418, cat# 17-0114-81, eBioscience), B220-APC (clone RA3-6B2, cat# 20-0452-U025, TONBO biosciences), F4/80-PerCP/Cy5.5 (clone BM8, cat# 123128, BioLegend), CD8α-PerCP/Cy5.5 (clone 53-6.7, cat# 100734, BioLegend), Ly6G-PE/Cy7 (clone 1A8, 127618, BioLegend), CD4-PE/Cy7 (clone GK1.5, cat# 100422, BioLegend), CD45.2-Pacific Blue (clone 104, cat# 109820, BioLegend), anti-CD16/32 (clone 93, cat# 101302, BioLegend), PD-1-APC (clone 29F.1A12, cat# 135209, BioLegend), and PD-L1-APC (clone 10F.9G2, cat# 124311, BioLegend).

### 1-4. Preparation of culture supernatant

A culture supernatant was prepared according to the previous report (Non Patent Literature 5). Specifically, SL4 cells were incubated with indomethacin (10 µM) overnight for elimination of PGE2 (prostaglandin E2), and were then suspended in PBS at a concentration of 10⁸ cells/ml. Thereafter, the cells were subjected to freeze-and-thaw cycles 5 times, using a 37°C constant-temperature bath and liquid nitrogen. After that, the cells were centrifuged at 8,000 × g for 5 minutes, and necrotic cell debris were removed. A culture supernatant was obtained by filtration through a 0.45 µm membrane filter (Millipore).

### 1-5. Reagents

LPS (O55:B5) was purchased from SIGMA-Aldrich (cat# L2637). Oligonucleotides were purchased from FASMAC. Indomethacin was purchased from WAKO (cat# 093-02473). Recombinant IL-1α was purchased from Peprotech (cat# 211-11A). Recombinant TCTP was purchased from ORIGENE (cat# TP301664). TUNEL staining was carried out using *in situ* Apoptosis Detection Kit (cat# MK500, TaKaRa). The endotoxin level of the recombinant TCTP was assessed using LAL Endotoxin Assay Kit, Chromogenic, ToxinSensor (cat# L00350, Genscript), and the endotoxin level was confirmed to be < 0.1 EU/µg.

### 1-6. Identification of TCTP

A culture supernatant from necrotic cells was first treated with a DNaseI solution (0.5 U/µl; TaKaRa Bio), RNaseA (0.25 mg/ml; MACHEREY-NAGEL), or PBS, and was then incubated at 37°C for 30 minutes. Regarding a proteinase K treatment, the culture supernatant was treated with proteinase K (100 µg/ml; TaKaRa Bio) at 37°C for 1 hour. Thereafter, the protease K-treated sample was treated with APMSF (5 mM; Nacalai Tesque, Inc.) on ice for 20 minutes so as to inactivate the proteinase K. Since the induction of the cytokine mRNA was abolished only by the proteinase K treatment, it was suggested that the activator be a protein. Subsequently, the culture supernatant was subjected to ion exchange chromatography (Hitrap Capto S column (cat# 17544105, GE healthcare) or Hitrap Capto Q column (cat# 11001302, GE healthcare)). The Hitrap Capto S column or the Hitrap Capto Q column was first washed with 1 ml of DDW, and was then equilibrated with 30 ml of PBS. Thereafter, the column was charged with 5 ml of a culture supernatant of SL4 cells, and was then washed with 5 ml of PBS. Flow-through was recovered, and PECs were then added thereto. As a result of an RT-qPCR analysis, it was found that the flow-through of the Hitrap Capto S column induces the cytokine mRNA, but that the flow-through of the Hitrap Capto Q column does not induce it. These results suggest that the molecule(s) as identification targets bind to the Hitrap Capto Q column.

Based on the above-described results, the following purification procedures, in which ion exchange chromatography was combined with size exclusion chromatography, were carried out. First, the Hitrap Capto S column was washed with 1 ml of DDW, and was then equilibrated with 30 ml of PBS. The column was charged with 5 ml of a culture supernatant of SL4 cells, and was then washed with 5 ml of PBS. Flow-through was recovered, and was then loaded on a Hitrap Capto Q column that had been equilibrated as in the case of the Hitrap Capto S column. Subsequently, the column was washed with 20 ml of PBS, and was then eluted with 5 ml of 0.4 M NaCl. The eluent was concentrated by Spin-X UF 10k MWCO (cat# CLS431488, MERCK). Finally, size exclusion chromatography was performed using a Superdex 200 Increase 10/300 GL column connected with AKTA purifier (GE Healthcare). The column was equilibrated with 2-fold column volumes of PBS, and the above concentrated eluent was loaded onto the column. Eluted droplets (18 droplets) were continuously recovered into wells of a 48-well plate, using AC-5700P MicroCollector (ATTO). Each fraction was added to PECs or RAW264.7 cells, and Cxcl1, Cxcl2,Tnf and Il1b mRNA levels were determined by RT-qPCR. The 18th to 27th fractions were subjected to silver staining with SilverQuest (cat# LC6070, Invitrogen), and the densest band that was correlated with cytokine inducing activity was subjected to LC-MS (liquid chromatography-mass spectrometry).

### 1-7. RT-qPCR

Total RNA was extracted from tissues or cells, using NucleoSpin RNA II (MACHEREY NAGEL), and was then reverse-transcribed using PrimeScript RT Master Mix (TaKaRa Bio). An RT-qPCR reaction was performed on LightCycler 480 (Roche Life Science) or LightCycler 96 (Roche Life Science), using the TB Green^{®} Premix Ex Taq^{™} II (TaKaRa Bio). The obtained values were normalized with respect to the expression level of Gapdh mRNA. Primers having the following sequences were used.

### Gapdh

Forward: 5'-ctcatgaccacagtccatgc-3' (SEQ ID NO: 26)
Reverse: 5'-cacattgggggtaggaacac-3' (SEQ ID NO: 27)

### Tnf

Forward: 5'-tcataccaggagaaagtcaacctc-3' (SEQ ID NO: 28)
Reverse: 5'-gtatatgggctcataccagggttt-3' (SEQ ID NO: 29)

### Cxcl1

Forward: 5'-agaccatggctgggattcac-3' (SEQ ID NO: 30)
Reverse: 5'-agcttcagggtcaaggcaag-3' (SEQ ID NO: 31)

### Cxcl2

Forward: 5'-tccagagcttgagtgtgacg-3' (SEQ ID NO: 32)
Reverse: 5'-tcagttagccttgcctttgttc-3' (SEQ ID NO: 33)

### Cxcr2

Forward: 5'- gacaccctcatgagaaccaagc-3' (SEQ ID NO: 34)
Reverse: 5'- gttaaggcagctgtggaggaag-3' (SEQ ID NO: 35)

### Il1b

Forward: 5'-gtggaccttccaggatgagg-3' (SEQ ID NO: 36)
Reverse: 5'-cggagcctgtagtgcagttg-3' (SEQ ID NO: 37)

### 1-8. CRISPR/Cas9

TCTP KO SL4 cells, TCTP KO B16F10 cells, and TCTP KO Meth-A cells were produced by CRISPR/Cas9 genome editing using the CRISPR design tool (http://www.genome-engineering.org, accessed May 2017). The genomic sequences of the TCTP gene (5'- CGGGCGGAAAAGGCCGACGC-3' (SEQ ID NO: 38) and 5'-AGGCCCGCCATTTCCCGCGC-3' (SEQ ID NO: 39)) were targeted. The first exon of the TCTP gene was flanked by the above two sequences, SEQ ID NO: 38 and SEQ ID NO: 39. Oligonucleotides corresponding to these guide sequences were cloned into the BbsI sites of pSpCas9(BB)-2A-GFP (PX458) (Addgene) and pSpCas9(BB)-2A-Puro (PX459) V2.0 (Addgene), respectively. The expression vector constructs were both introduced into SL4 cells. The construct-introduced cells were first selected with puromycin, and were then subjected to single cell sorting using FACSAria II (BD Biosciences) or SH800S (SONY).

### 1-9. immunoblotting

Immunoblot analysis was performed according to the previous report (Inoue et al., Nature 434, 243-249, doi: 10.1038/nature03308 (2005)). β-Actin (cat# A5441) was purchased from Sigma-Aldrich. TCTP antibodies (cat# ab133568 and cat# ab37506) were purchased from Abcam. TCTP antibody (cat# 5128S) was purchased from Cell Signaling Technology. β-Actin was used as a loading control. In order to determine the amount of TCTP in serum, the serum was subjected to immunoblot analysis using a TCTP antibody (ab133568). Recombinant TCTP (cat# TP301664, OriGene) was used in the measurement of the TCTP amount. Anti-rabbit IgG-HRP (cat# NA934V, GE Healthcare) or anti-mouse IgG-HRP (cat# NA931V, GE Healthcare) was used as a secondary antibody. Immunoblotting signals were detected by FUSION Solo S (Vilber-Lourmat), and were then analyzed by FUSION Capt Advanced software (Vilber-Lourmat).

### 1-10. In vitro cell proliferation analysis

WT SL4 cells and TCTP KO SL4 cells (2.5 × 10⁵ cells), B16F10 cells (1.2 × 10⁵ cells), or Meth-A cells (2.5 × 10⁵ cells) were seeded in each well of a 6-well plate. Every 3 days, one eighth of the cells were passaged to a new 6-well plate. The number of the cells was calculated on Days 3, 6, and 9.

### 1-11. Proliferation assay of subcutaneously transplanted tumor

2 × 10⁵ SL4 cells, 1 × 10⁵ B16F10 cells, or 5 × 10⁵ Meth-A cells were transplanted into the subcutis of mice. The tumor volume was calculated according to the equation: average volume = πab²/6 (wherein a and b indicate the major axis and the minor axis, respectively).

### 1-12. Apc^{+/Δ716} colorectal cancer models

TCTP^{flox/flox}, Apc^{+/Δ716} mice or TCTP^{flox/flox}, Apc^{+/Δ716}, Villin-CreERT2 mice were treated with tamoxifen at a dose of 4 mg/mouse once a week, starting from 5 weeks of age. Intestinal tumors of these mice were analyzed using stereoscope (Leica S9 D, Leica), when the mice were 10-week-old.

### 1-13. Establishment of TCTP-introduced cell line

DNA encoding the human IL-2 peptide (MYRMQLLSCIALSLALVTNS: SEQ ID NO: 40) was fused with the 5'-terminus of mouse TCTP cDNA. IL-2ss-TCTP and WT TCTP cDNA fragments were each inserted into a pMXs-IRES-GFP retroviral expression vector. Introduction of the vector into TCTP KO SL4 cells by retrovirus was carried out according to the previous report (Chiba et al., Elife. 2014 Aug 22; 3:e04177. doi: 10.7554/eLife.04177.). Thereafter, GFP-positive cells were sorted using Cell Sorter SH800S (SONY). Mock cells or IL-2ss-TCTP-introduced cells were identified by performing the aforementioned immunoblotting analysis on a culture supernatant recovered from a 60-mm culture dish, on which 2 × 10⁶ cells had been seeded.

### 1-14. Enzyme-Linked Immunosorbent assay (ELISA)

A tumor was excised on Day 21 after the transplantation, and was then finely minced in a lysis buffer (20 mM Tris-HCl, 150 mM NaCl, 1 mM EDTA, 1 % Triton X-100, 1 mM Na₃VO₄, and 1 mM APMSF). Thereafter, the resultant tumor was incubated on ice for 30 minutes, a lysate was then centrifuged, and a supernatant was then recovered for use in ELISA. The amounts of mouse CXCL1 and CXCL2 generated in TIME were quantified using an ELISA kits (R&D Systems) according to an instruction manual included therewith.

### 1-15. Evaluation of immunosuppressive activity of PMN-MDSCs

T cells were collected from the splenocytes of non-tumor bearing mice, using Pan T cell isolation kit II (Miltenyi Biotec). The prepared cells were stained with CFSE, using CellTrace^{™} CFSE Cell Proliferation Kit (Thermo Fischer Scientific) according to an instruction manual included therewith. PMN-MDSCs were collected from the splenocytes of mice on Days 17-19 after subcutaneous transplantation of SL cells (2 × 10⁵ cells), using Ani-Ly6-G MicroBeads (Miltenyi Biotec). Neutrophils were collected from non-tumor bearing C57BL/6 mice by the same method as that for PMN-MDSCs. CFSE-labelled T cells (1 × 10⁵ cells) and PMN-MDSCs (5 ×10⁴ cells, 2.5 ×10⁴ cells, or 1.25 ×10⁴ cells) were seeded in a 96-well plate. Proliferation of T cells was induced for 3 days, using Dynabeads Mouse T-Activator CD3/CD28 (Thermo Fischer Scientific), and thereafter, a flow cytometry analysis was carried out to evaluate a CFSE dilution rate in the T cells.

### 1-16. In vivo depletion of CD8⁺ T cells, NK cells and PMN-MDSCs

For depletion of NK cells, anti-asialo GM1 (cat# 014-09801, WAKO) or rat control IgG (cat# 31933, Thermo Fisher) was intraperitoneally transplanted into the mice (200 µg/mouse) on Days 1, 3, 7, 11, and 15 after the transplantation of the tumor cells. For depletion of CD8⁺ T cells, anti-CD8α (clone 2.43, cat# BE0061, Bio X Cell) or rat control IgG (cat# 31933, Thermo Fisher) was intraperitoneally transplanted into the mice (100 µg/mouse) on Days 1, 3, 7, and 11 after the transplantation of the tumor cells. For depletion of NK cells and CD8+ T cells, anti-asialo GM1 (cat# 014-09801, WAKO) or rat control IgG (cat# 31933, Thermo Fisher) was intraperitoneally transplanted into RAG1 KO mice (200 µg/mouse). For depletion of PMN-MDSCs, an anti-Ly6G antibody (cat# BE0075-1, Bio X Cell) or rat control IgG was intraperitoneally transplanted into the mice on Days 1, 3, 5, 7, 9, and 11 after the transplantation of the tumor cells.

### 1-17. Dihydroartemisinin (DHA) or o-vanillin treatment

DHA (Selleck, TX, USA) was dissolved in DMSO, and was then administered intraperitoneally (50 mg/kg) into the mice every day, starting from Day 1 of the tumor transplantation. Regarding combined administration of an anti-PD-1 antibody and DHA, the DNA administration was terminated on Day 6. O-vanillin (cat# 120804-10G, Merck) was first dissolved in DMSO to a concentration of 500 mg/ml, and was then diluted with PBS to a final concentration of 50 mg/ml. The diluted solution was orally administered (50 mg/kg) to the mice every other day, starting from Day 1 of the tumor transplantation.

### 1-18. Preparation of monoclonal antibodies

### 1-18-1. Preparation of antibodies

Mouse monoclonal antibodies reacting against TCTP were prepared according to a standard hybridoma technology by MAB Institute, Inc. (http://www.monoclo.com; Nagano, Japan). BALB/c mice were immunized with a synthetic peptide (EDGVTPYMIFFKDGLEMEKC; SEQ ID NO: 25) that is a partial sequence of human TCTP. Antibodies were screened based on an immunoblotting analysis and an immunoprecipitation analysis against TCTP. As a result, antibodies 55F3, 44E1 and 51A9 were obtained. Regarding the treatment using an antibody, 55F3 or control IgG (cat# 0107-01, SouthernBiotech) was intraperitoneally transplanted into the mice at a dose of 200 µg per mouse.

### 1-18-2. Determination of amino acid sequences of variable regions of antibody heavy chain and light chain

The amino acid sequences of the heavy chain and light chain of the prepared monoclonal antibodies were determined with reference to PLoS ONE e0218717,14: 2019.

Total RNA was extracted from hybridoma cells, and cDNA was then synthesized using RT (reverse transcription) primers specific to the variable regions of the antibody heavy chain and light chain (mIGK RT, mIGHG RT, and Template-switch oligo F). Subsequently, the prepared cDNA was used as a template, and a PCR reaction was then carried out using specific primers (ISPCR, mIGK PCR, and mIGHG PCR). The obtained cDNA was subjected to TA cloning (pTA2 vector, TOYOBO) for sequencing.

Detailed experimental conditions are as follows.

### (1) Preparation of cDNA

### Primers used in reverse transcription reaction

Template-switch oligo F: 5'-aagcagtggtatcaacgcagagtacatGGG-3' (G: riboguanine) (SEQ ID NO: 41)
mIGK RT: 5'-ttgtcgttcactgccatcaatc-3' (SEQ ID NO: 42)
mIGL RT: 6'-ggggtaccatctaccttccag-3' (SEQ ID NO: 43)
mIGHG RT: 5'-agctgggaaggtgtgcacac-3' (SEQ ID NO: 44)

### Reaction solution (I) (cDNA synthetic reaction)

2 µL of 50 ng/µL total RNA,
1 µL of 10 µM primer for reverse transcription reaction (mIGK RT, mIGL RT, or mIGHG), and
1 µL of 10 mM dNTPs.

These substances were mixed with one another in a 8-well strip tube.

### Reaction solution (II) (cDNA synthetic reaction)

2.2 µL of H₂O,
2 µL of 5 × SMARTScribe buffer,
1 µL of 20 mM DTT,
0.3 µL of 100 µM template-switch oligo F, and
0.5 µL of 100 U/µL SMARTScribe Reverse Transcriptase.

These substances were mixed with one another in a 8-well strip tube.

The reaction solution (I) was subjected to a heat treatment using a thermal cycler at 72°C for 3 minutes. After completion of the heat treatment, 6 µL of the reaction solution (II) was added to the reaction solution (I), and the thus mixed solution was then incubated using a thermal cycler at 42°C for 60 minutes. Thereafter, the reaction mixture was subjected to a heat treatment at 70°C for 5 minutes, and the reaction was then terminated.

### (2) Determination of amino acid sequences of variable regions of antibody heavy chain and light chain

The synthesized cDNA was used as a template, and a PCR reaction was carried out using the following primers. Using the obtained amplified product, the DNA sequences of the variable regions of the antibody heavy chain and light chain, and thereafter, amino acid sequences encoded thereby were determined.

### Primers

ISPCR F: 5'-aagcagtggtatcaacgcagag-3' (SEQ ID NO: 45)
mIGK PCR: 5'-acattgatgtctttggggtagaag-3' (SEQ ID NO: 46)
mIGL PCR: 5'-atcgtacacaccagtgtggc-3' (SEQ ID NO: 47)
mIGHG PCR: 5'-gggatccagagttccaggtc-3' (SEQ ID NO: 48)

### Reaction solution

25 µL of 2 × PCR buffer for KOD FX
10 µL of 2 mM dNTPs
3 µL of Synthesized cDNA from the RT reaction
2.5 µL of 10 µM universal forward primer ISPCR
2.5 µL of 10 µM reverse PCR primer (mIGK PCR, mIGL PCR, or mIGHG PCR)
6 µL of H₂O
1 µL of KOD FX (1 U/µL)

### PCR Cycle

After a reaction performed at 98°C and 30 seconds,
98°C, 15 seconds,
63°C to 57.5°C, 30 seconds (the temperature was decreased by 0.5°C in each cycle), and
72°C, 30 seconds.

The aforementioned reaction was carried out for 10 cycles, and further, a reaction consisting of:
98°C, 15 seconds,
56°C, 30 seconds, and
72°C, 30 seconds,
was carried out for 15 cycles.

Thereafter, a reaction was carried out at 72°C for 7 minutes, and the reaction mixture was then left at rest at 4°C.

The determined amino acid sequences of the variable regions of the antibody heavy chain and light chain were matched with the antibody sequence database (http://www.abybank.org/kabat/ and http://www.bioinf.org.uk/abs/info.html#cdrid, etc.), and amino acid sequences corresponding to CDRs according to Kabat definition were specified.

1-19. Analysis of data obtained from patients with colorectal cancer

The TCGA dataset of 640 colorectal cancer patients was downloaded from the cBioPortal (https://www.cbioportal.org/, accessed December 2019). Then, GISTIC 2.0 analysis was carried out in the cBioportal platform.

### 1-20. Pathological analysis

SL4 tumor and Apc^{Δ716} mouse tumor-derived large intestine were preserved in PBS containing 4% paraformaldehyde, and were then embedded in paraffin. Hematoxylin and eosin (H&E) staining, TUNEL staining, and 3,3'-diaminobenzidine (DAB) staining were carried out on CD31 (cat# ab28364, Abcam) or Ly6G (cat# 127601, Biolegend) at the core laboratory for pathological analysis of the Institute of Medical Science, the University of Tokyo. Formalin fixed paraffin embedded tissues of the large intestines of colorectal cancer patients and normal large intestine epithelial samples of the same patients were prepared and analyzed at Kanazawa University. This analysis was approved by Human Genome/Gene Analysis Research Ethics Committee of Kanazawa University (2016-086-433), and written consent was obtained from the patients.

TCTP (cat# 133568, Abcam) or CD15 (cat# M363129, Dako) was used in DAB staining. Briefly, a paraffin-embedded sample was deparaffinized by incubation with xylene two times, and was then dehydrated with a series of ethanol, followed by rehydration in PBS. Heat-induced epitope retrieval was performed using an antigen retrieval reagent pH9 (cat# 415211, NICHIREI BIOSCIENCES INC.) at 121°C for 10 minutes. In order to deactivate endogenous peroxidase, REAL Peroxidase-Blocking solution (cat# S2023, DAKO) was used according to an instruction manual included therewith, followed by blocking with 1% BSA/TBST at room temperature for 30 minutes. Sections were incubated together with a TCTP antibody or a CD15 antibody at room temperature for 1 hour. Detection of the primary antibody was performed using Histofine simple stain MAX-PO (MULTI) (cat# 424151, NICHIREI BIOSCIENCES INC.), and using DAB (cat# 415171, NICHIREI BIOSCIENCES INC.) as a substrate. Samples were counterstained with hematoxylin (cat no. 415081, NICHIREI BIOSCIENCES INC.). Quantification of signal intensity from TCTP was performed according to the previous report, using ImageJ Fiji (Bio Protoc. 2019 Dec 20; 9(24): e3465.). The average intensity in normal colonic mucosa was set as 1. Quantification of CD31-positive areas was performed using a BZ-9000 microscope and a cell count software BZ-H4C (Keyence).

### 1-21. Microarray

PECs were stimulated with a culture supernatant of necrotic cells (2 × 10⁶ SL4 cells). After incubation for 2 hours, total RNA was extracted and was then subjected to an analysis with Clariom S Array (Thermo Fisher Scientific). Volcano plot was produced by Transcriptome Analysis Console (TAC) software v4.0 (Thermo Fisher Scientific). Microarray data were registered in the Gene Expression Omnibus (GEO) database (Accession No. GSE150465).

### 1-22. Immunostaining

Cells were cultured on a 35 mm glass-bottom dish (MATSUNAMI) overnight, and thereafter, the cultured cells were washed with PBS and were then fixed with 4% paraformaldehyde/PBS for 15 minutes. After washing with PBS, the resulting cells were permeabilized with 0.5% Triton X-100/PBS for 15 minutes, and were then blocked with 3% BSA/PBS. Subsequently, the cells were incubated together with the anti-TCTP antibody (ab37506) for 2 hours, followed by washing, and the resulting cells were treated with a secondary antibody (Alexa Fluor 594 Goat anti-rabbit IgG, cat# A-11012, Invitrogen) for immunostaining. After washing with PBS, nuclear counter-staining was carried out using VECTASHIELD Hard Set Mounting Medium with DAPI (cat# H-1500, VECTOR LABORATORIES, INC), and immediately thereafter, an analysis was carried out by a C2si confocal microscopy system (NIKON) equipped with ECLIPSE Ti microscopy (NIKON).

### 1-23. Adoptive transfer of PMN-MDSCs

Bone marrow cells and splenocytes were collected from (tumor-bearing) mice on Day 17 after subcutaneous transplantation of SL4 cells (2 × 10⁵ cells). Ly-6G⁺ cells were separated using anti-Ly6-G MicroBead Kit, mouse (Miltenyi Biotec). The separated Ly-6G⁺ cells (4 × 10⁶ cells) were intravenously injected into mice on Days 1, 4, 7, 10, and 13 after subcutaneous transplantation of TCTP KO SL4 cells (2 × 10⁵ cells).

### 1-24. immunoprecipitation

HEK293T cells (5 × 10⁶ cells) were seeded, and were transiently transfected with only pCXNII-FLAG-hTCTP (2 µg), or with pCXNII-FLAG-hTCTP (2 µg) in combination with pcDNA3.1-hTLR2-YFP (2 µg), using X-tremeGENE9 (Roche Life Science). A pcDNA3.1-hTLR2-YFP vector was kindly provided by Dr. Douglas Golenbock.

Thereafter, a cell lysate was prepared using a lysis buffer (20 mM Tris-HCL (pH 7.5), 150 mM NaCl, 1 mM EDTA, 1% Triton X-100, and 1 mM PMSF). The cell lysate (1 mg) was subjected to immunoprecipitation using 1 µg of an anti-GFP antibody (598; MBL) and 30 µl of Dynabeads Protein G for immunoprecipitation (Thermo Fisher Scientific). Thereafter, the obtained immunoprecipitate was subjected to immunoblotting using an anti-GFP antibody or an anti-FLAG M2 antibody (Sigma Aldrich).

### 1-25. Luciferase reporter assay

Mouse TLR3, TLR7, TLR9, and human CD14 cDNAs were cloned into a pCXNII-HA vector. A Human TLR2 cDNA construct (pcDNA3.1-hTLR2-YFP), a mouse TLR3 cDNA construct (pCXNII-HA-mTLR3), a mouse TLR7 cDNA construct (pCXNII-HA-mTLR7), or a mouse TLR9 cDNA construct (pCXNII-HA-mTLR9), together with an NFκB luciferase reporter (pNFκB-Luc (Stratagene)), was transfected into HEK293T cells. As for TLR2 stimulation, a human CD4 cDNA construct (pCXNII-HA-hCD14) was co-transfected into the cells. Twenty-four hours after the transfection, 2 × 10⁴ cells were seeded in a 96-well dish. Twenty-four hours after the seeding, the cells were treated with recombinant TCTP or each TLR agonist. Thereafter, luciferase activity was measured with Pikka Gene Dual Assay kit (cat# PD-11, TOYO B-Net), using MicroLumat Plus LB96V (Berthold Technologies) according to an instruction manual included therewith.

### 1-26. Quantification of G-CSF and GM-CSF

Quantification of G-CSF (cat# 560152, BD Biosciences) and GM-CSF (cat# 558347, BD Biosciences) was performed using cytometric bead assay (CBA) according to an instruction manual included therewith.

### 1-27. Induction of cell death

In order to induce apoptosis, serum starvation and adriamycin treatment were carried out. For the serum starvation, SL4 cells (5 × 10⁴ cells) were seeded in a 48-well dish, and twelve hours after the seeding, the medium was replaced with a serum-free medium. Seventy-two hours after the serum starvation, a culture supernatant was recovered. For the adriamycin treatment, SL4 cells were seeded in the same manner as that for the serum starvation. Twelve hours after the seeding, the cells were treated with a medium containing adriamycin (50 µM) for 24 hours, and thereafter, a culture supernatant was recovered.

In order to induce necrosis, SL4 cells (1 × 10⁷ cells) were suspended in 1 ml of PBS. Freezing and thawing were performed as described in the above "1-4. Preparation of culture supernatant." As a control, a supernatant of SL4 cells incubated in PBS for the same period of time as that for the freezing and thawing was used. Hypoxic treatment (1% O₂, 5% CO₂) was performed using a multi-gas incubator (MCO-5MUV-PJ, Panasonic).

### 1-28. Quantification of TCTP in human serum

Human sera of colorectal cancer patients and non-cancer patients were obtained from Biobank Japan. The present study was approved by the institutional ethics committee of the University of Tokyo (20-239). Quantification of TCTP was carried out by immunoblotting.

### 1-29. Administration of dead tumor cells to subcutaneous tumor models

TCTP WT cells or TCTP KO cells on a 10 cm dish were lethally irradiated with X-ray (100 Gy). TCTP WT SL4 cells (2 × 10⁵ cells) were mixed with equal amounts of the above lethally irradiated TCTP WT cells or TCTP KO cells, and the thus obtained mixture was subcutaneously transplanted into C57BL/6 cells.

### 1-30. Statistical processing

Sample size and statistical tests were carried out as described in Description of the Drawings. Data were expressed as a mean value ± standard deviation (s.e.m.), unless otherwise specified. One-way ANOVA with Dunnett's or Tukey's multiple comparisons test, Spearman correlation coefficients calculation, Log-rank test and two-sided Student's t-test were performed using Prism 8.0 (GraphPad Software). All alpha levels were 0.05, with P < 0.05 considered a significant difference.

### 2. Results

### 2-1. Identification of tumor cell-derived TCTP as immunomodulator

Since a considerable number of tumor cells die during tumor proliferation (mostly, due to necrosis), we made a working hypothesis that the environment of the necrotic cells may function as an immunomodulator for MDSCs (myeloid-derived suppressor cells; i.e. bone marrow-derived immunosuppressor cells) in the tumor immune microenvironment (TIME). As an approach to identify a dead tumor cell-derived immunomodulator, a culture supernatant model of necrotic SL4 cells (a mouse colorectal cancer cell line) was first used (Non Patent Literature 5) (Figure 1(a)). When an SL4 cell culture supernatant was exposed to mouse PECs, the mRNA expression of multiple cytokines was induced. These mRNAs were analyzed by microarray (Figure 1(b)) and RT-qPCR (Figure 1(c)). Among cytokines in which induction of the mRNA expression was confirmed, Cxcl1 and Cxcl2 have been known to regulate migration of hematopoietic cells such as MDSCs. Thus, we focused on Cxcl1 and Cxcl2. CXCR2 that is a receptor common in CXCL1 and CXCL2 has been known to be critical for the migration of PMN-MDSCs in mice (Non Patent Literature 6).

In order to identify chemokines derived from dead tumor cells, which induce the expression of Cxcl1 and Cxcl2, molecules of interest were attempted to be isolated from a culture supernatant of SL4 cells, by applying ion exchange chromatography and size exclusion chromatography. The culture supernatant of SL4 cells was passed through an anion-exchange column (Hitrap Q) and a cation-exchange column (Hitrap S). Flow-through was recovered from the Hitrap S column, and was then loaded on the Hitrap Q column. A fraction binding to Hitrap Q was recovered, and was then loaded on the size exclusion column (Superdex 200). The obtained fractions were each added to PECs (Cxcl1, Cxcl2, and Il1b; 2 × 10⁵ cells) or RAW264.7 cells (Tnf; 2 × 10⁵ cells), and were then incubated for 2 hours. Cxcl1, Cxcl2, Tnf, and Il1b mRNAs were quantified by an RT-qPCR method. The peak fraction of each mRNA expression level was subjected to SDS-PAGE and sliver staining, and thereafter, liquid chromatography mass spectrometry (LC-MS) was carried out on the most abundant protein. As a result, it was revealed that TCTP (Tumor cell-derived translationally controlled tumor protein) would be a candidate inducer of the above-described each mRNA. It has been reported that TCTP is present in the cytoplasm of eukaryotic cells (Non Patent Literature 7, Non Patent Literature 8, and Non Patent Literature 9). However, the function of TCTP that is present outside of the cells has not been known so far.

PECs were stimulated with recombinant TCTP, and the expressed mRNA was then quantified by RT-qPCR. As a result, it was observed that the mRNA expression of Cxcl1 and Cxcl2 and other cytokines (Tnf and Il1b) was induced (Figure 2(a)). From these results, it was confirmed that TCTP has immunostimulatory activity. Interestingly, it has been reported that the expression level of TCTP mRNA is increased in tumor samples (Du et al., Oncotarget 8, 101922-101935, doi: 10.18632/oncotarget.21747 (2017)). However, whether or not TCTP functions in tumor progression, and if TCTP functions in tumor progression, how TCTP functions, has not yet been elucidated.

TCTP was abundantly present in a culture supernatant of dead tumor cells (tumor dead cells), whereas living tumor cells (tumor living cells) hardly released TCTP (Figure 3(a)). Furthermore, as shown in Figure 2(b), serum TCTP protein levels were increased over time in C57BL6 mice subcutaneously transplanted with SL4 cells. These results suggested that TCTP be released from dying tumor cells *in vivo.* Likewise, an increase in the TCTP protein levels was observed also in the sera of mice bearing B16F10 melanoma and Meth-A fibrosarcoma tumors (Figure 3(b)).

### 2-2. Contribution of tumor-derived TCTP to tumor growth

Taking into consideration the above-described results, the influence of TCTP on proliferation of tumors formed by TCTP-expressing SL4 cells and TCTP-deficient SL4 cells was studied. Whole lysates of TCTP-expressing SL4 cells (TCTP WT SL4: WT) and TCTP-deficient SL4 cells (TCTP KO SL4: KO) were prepared, and immunoblotting was then performed on TCTP and β-actin, so that it was confirmed that TCTP was deleted in TCTP KO SL4 (Figure 3(c)). The proliferation speed of TCTP WT SL4 cells was equivalent to that of TCTP KO SL4 cells, *in vitro,* under standard conditions, under hypoxic conditions, and under low serum conditions (Figures 3(d) and (e)). In contrast, when TCTP WT SL4 cells and TCTP KO SL4 cells were each transplanted into C57BL/6 mice via subcutaneous injection and the volume of a tumor generated *in vivo* was then measured over time, it was found that proliferation of a TCTP KO SL4 cell-derived tumor became significantly slow, compared with proliferation of a TCTP WT SL4 cell-derived tumor (Figure 2(c)). Moreover, when a TCTP gene was introduced again into TCTP KO SL4 cells and a TCTP protein was allowed to express again, it was confirmed that the proliferation speed of a tumor *in vivo* was recovered (Figure 3(f)). Furthermore, the TCTP genes ofB 16F 10 cells and Meth-A cells were destroyed (Figure 3(c)), and the same studies as those described above were then carried out. As a result, as in the case of SL4 cells, the proliferation speed of a tumor derived from a wild-type strain *in vitro* was equivalent to the proliferation speed of a tumor derived from a TCTP gene-deficient strain *in vitro* (Figures 3(g) and (h)). On the other hand, with regard to proliferation a tumor transplanted into the living body of mice, proliferation of a TCTP gene-deficient strain-derived tumor became significantly slow, compared with proliferation of a wild-type strain-derived tumor (Figures 2(d) and (e)).

The aforementioned results show that TCTP promotes proliferation of a tumor *in vivo.*

Whether or not dead tumor cell-derived TCTP promotes tumor growth was studied. When TCTP WT SL4 cells (dead WT cells) irradiated with a lethal amount of X-ray were administered to mice, it promoted the growth the TCTP WT SL4 cell-derived tumor. On the other hand, when TCTP KO SL4 cells (dead KO cells) irradiated with a lethal amount of X-ray were administered to mice, it did not promote the growth of a tumor (Figure 4(c)).

Further, in order to reveal the role of TCTP in tumor progression, colorectal tumor models promoted by deletion mutation (Apc^{Δ716}) of an Apc gene as a tumor suppressor were studied in the presence or absence of a TCTP gene (Figure 2(f)). TCTP^{flox/flox}, Apc^{+/Δ716}, villin-Cre ERT2 mice, in which the expression of a TCTP gene can be regulated by tamoxifen, were used (Figure 2(f)). In these mice, deletion of the TCTP gene is generated in intestinal epithelial cell-specific manner by administration of tamoxifen. As a result, when tamoxifen-treated mice (TCTP-deficient mice) were compared with tamoxifen-non-treated mice (mice having a TCTP gene) in terms of a total number of tumors generated in the intestines, a significant difference was not found between them (Figure 4(a), Total). On the other hand, the number of tumors having a tumor diameter of more than 2.0 mm was significantly reduced in the TCTP-deficient mice (Figure 4(a), 2.0 mm < ). These results show that TCTP promotes tumor proliferation, although it does not have influence on the occurrence rate (frequency of occurrence) of tumors. These results correspond to the aforementioned experimental results obtained using cancer cell-transplanted mouse models. Specifically, the results mean that TCTP is not associated with the occurrence rate of tumors but it promotes proliferation of generated cancers.

### 3. Studies regarding role of extracellular TCTP

Next, whether or not TCTP released to the outside of the cells promotes tumor proliferation was studied. For extracellular secretion of TCTP, using a retroviral gene transfer vector, cDNA encoding a chimeric TCTP protein fused with a human IL-2 signal sequence was allowed to express in TCTP KO SL4 cells, and the resulting cells (IL-2ss-TCTP SL4 cells) were then allowed to proliferate in a cell medium. A TCTP protein was detected *in vitro* in a culture supernatant (Figure 5(a)), and was not detected in the cells (Figure 6(a)). Subsequently, PECs were stimulated with a culture supernatant of TCTP KO SL4 cells and a culture supernatant of IL-2SS-TCTP SL4 cells, and then, whether or not induction of cytokines was observed therein was examined. Consequently, an increase in the expression levels of Cxcl1 and Cxcl2 mRNAs was confirmed as a result of stimulation with the culture supernatant of IL-2SS-TCTP SL4 cells (Figure 6(b), IL2ss-TCTP). The proliferation speed of IL-2ss-TCTP SL4 cells *in vitro* was equivalent to the proliferation speed of TCTP KO SL4 cells *in vitro* (Figure 5(b)). However, the proliferation speed of IL-2ss-TCTP SL4 cell-derived tumors *in vivo* was significantly quicker than the proliferation speed of TCTP KO SL4 cell-derived tumors *in vivo* (Figure 5(c)).

From the above results, it was demonstrated that TCTP released to the outside of the cells functions as an immunomodulator, and functions as a factor of promoting tumor proliferation *in vivo.*

Considering the above-obtained results, it is conceived that TCTP released from dying tumor cells would induce CXCL1 and CXCL2, which would induce PMN-MDSCs to TIME and would suppress antitumor immune response in the TIME, so that they can promote tumor proliferation.

In fact, when the expression level of CXCL1 in TCTP KO SL4 tumors was compared with the expression level of CXCL1 in TCTP WT SL4 (TCTP-expressing SL4) tumors, the expression level of CXCL1 was significantly high in the TCTP WTSL4 tumors (Figure 5(d)). Similar results were found also in the case of CXCL2 (Figure 5(d)). Also, the expression level of CXCL1/2 in IL-2ss-TCTP tumors was significantly higher than the expression level of CXCL1/2 in TCTP KO SL4 tumors (Figure 5(e)). These results show that extracellular TCTP induces these cytokines in the TIME.

Next, tumor-infiltrating immune cells were prepared from TCTP WT SL4 tumors and TCTP KO SL4 tumors, and were then analyzed by flow cytometry. As shown in Figure 7(a)), it was confirmed that CD11b⁺Ly6C^{low}Ly6G⁺ cells indicating mouse PMN-MDSCs were drastically decreased in TCTO KO SL4 tumors. In contrast, CD11b⁺Ly6C^{high}Ly6G cells indicating M-MDSCs and bone marrow cells such as CD11b⁺Ly6C-Ly6G⁻F4/80⁺ tumor-associated macrophages (TAMs) did not show such drastic changes (Figures 7(a) and (b)). The amount of PMN-MDSCs was increased in IL-2ss-TCTP tumors, compared with in TCTP KO tumors, but there was almost no difference in terms of the number of M-MDSCs (Figure 7(c)). It is notable that there was no significant difference between WT tumors and TCTP KO tumors, in terms of the expression levels of G-CSF and GM-CSF promoting proliferation of MDSCs (Figure 6(c)). These results show that G-CSF and GM-CSF are not major causes of a reduction in the cell number of PMN-MDSCs in TCTP KO tumors. Similarly, a decrease in the cell number of PMN-MDSCs was also observed in B16F10 and Meth-A tumors (Figures 6(d) and (e)). Interestingly, a significant decrease in the number of ly6G⁺ cells indicating PMN-MDSCs was also observed in the de novo tumors of the aforementioned TCTP^{flox/flox}, Apc^{+/Δ716}, villin-Cre ERT2 mice that are tumor-transplanted models.

In order to confirm that the above CD11b⁺Ly6C^{low}Ly6G⁺ cells actually indicate PMN-MDSCs, CD11b⁺Ly6C^{low}Ly6G⁺ cells derived from SL4 tumor-bearing mice and CD11b⁺Ly6C^{low}Ly6G⁺ cells derived from mice that did not bear SL4 tumors were subjected to *in vitro* T cell proliferation assay. As shown in Figure 7(e), T cells were stimulated with anti-CD3/CD28 antibodies in the presence of the CD11b⁺Ly6C^{low}Ly6G⁺ cells separated from the SL4 tumor-bearing mice, and proliferation of the T cells was then observed. As a result, proliferation of the T cells was suppressed, depending on the abundance of the CD11b⁺Ly6C^{low}Ly6G⁺ cells. In contrast, when the same experiment was carried out using the CD11b⁺Ly6C^{low}Ly6G⁺ cells derived from the mice that did not bear SL4 tumors, the effect of suppressing proliferation of the T cells was not observed. Furthermore, proliferation of TCTP KO SL4 tumors was significantly increased by adoptive immunity with PMN-MDSCs collected from the SL4 tumor-bearing mice (Figure 6(g)). The aforementioned results suggest that retardation of proliferation of TCTP KO tumors be induced by PMN-MDSCs, and the results support the presence of a TCTP-PMN-MDSC pathway inducing progression of immunosuppressive TIME.

The results of the aforementioned MDSC adoptive immunity suggest that antitumor lymphocytes still activate even in mice bearing TCTP KO tumors. As shown in Figure 8(b), the number of CD8⁺ T cells in the TIME was significantly increased in TCTP KO SL4 tumor-bearing mice, compared with in TCTP WT SL4 tumor-bearing mice. On the other hand, the number of CD8⁺ cells was smaller in IL-2ss-TCTP tumors, than in TCTP KO tumors. In addition, intratumoral NK cell activation markers (CD69 and CD107a) were also increased in TCTP KO SL4 tumor-bearing mice, compared with in TCTP WT SL4 tumor-bearing mice (Figure 6(h)). Accordingly, these results suggest that both CD8⁺ T cells and NK cells be associated with induction of immune response to tumors.

In particular, proliferation of TCTP KO SL4 tumors was partially recovered by the removal of either CD8⁺ T cells or NK cells (Figures 8(c) and (d)), and proliferation of the tumors was promoted by the removal of both of the two types of cells (Figure 8(e)). The aforementioned observation results can be supported by the explanation that both the CD8⁺ T cells and the NK cells are present in a state in which they retain activity on TCTP KO tumors in the TIME as a result of deletion of MDSC repertoire. On the other hand, when the CD8⁺ T cells and the NK cells were allowed to disappear in WT tumors, it did not have influence on tumor proliferation. These results suggest that these ejector cells have already been in a dysfunctional state by stronger MDSC repertoire (Figures 8(c) to (e)). It is notable that, basically, no difference was found between the expression level of PD-L1 on TAMs, MDSCs and stroma cells, and the expression level of PD-1 on CD8⁺ T cells in TCTP WT tumors and TCTP KO tumors (Figures 9(a) and (b)). Moreover, the density of endothelial cells (CD31⁺ cells) in TCTP KO tumors was equivalent to the density thereof in TCTP WT tumors (Figure 9c).

The aforementioned results suggest that PMN-MDSCs that are incorporated into the TIME by TCTP released from tumor cells suppress the antitumor immunosuppressive action of CD8⁺ T cells and NK cells, and promote tumor proliferation, at least, in some parts.

### 4. Identification of cells and receptors, on which TCTP act

From the previous results, it is conceived that a TCTP-CXCL1/2-PMN-MDSC pathway is associated with suppression of antitumor immunity in the TIME (tumor immune microenvironment). Hence, next, the cell types that become factors of induction of chemokines in the TIME were studied. Subsets of immune cells were sorted from the TIME of TCTP WT SL4 tumors and the TIME of TCTP KO SL4 tumors, and the expression levels of chemokine mRNAs were then examined. As a result, D11b⁺Ly6C^{high}Ly6G cells (M-MDSCs) were found to express the highest level of Cxcl1 mRNA (Figure 10(a)). Notably, the expression level of Cxcl1 mRNA in M-MDSCs separated from TCTP KO SL4 tumors was significantly reduced (Figure 10(b)). In this respect, SL4 tumor cells did not respond to TCTP *in vitro* and did not induce the expression of Cxcl1 mRNA (Figure 11(a)). Moreover, *in vitro,* the expression level of Cxcl1 mRNA in TCTP WT SL cells was equivalent to that in TCTP KO SL4 cells (Figure 11(b)). Therefore, it is considered that tumor cells themselves hardly induce CXCL1 as a result of stimulation with TCTP. In addition, the expression of the mRNA of Cxcr2 as a receptor gene of CXCL1/2 chemokine was highest in PMN-MDSCs among all other cells (Figure 11(c)). These results suggest that TCTP act on M-MDSCs and induce the expression of CXCL1/2 chemokine, and as a result of stimulation with such CXCL1/2 chemokine, CXCR2-expressing PMN-MDSC cells migrate into the TIME, so that suppression of the antitumoral immune system takes place, thereby promoting tumor growth.

Next, whether or not TCTP-induced chemokine activates specific receptors was studied. First, the involvement of adaptor molecules that act on several innate immune receptors was examined. As shown in Figure 10(c), the TCTP-mediated chemokine induction was abolished in PECs deficient in a MyD88 gene, but was not abolished in PECs deficient in an IPS1/MAVS or STING gene. MyD88 is an adaptor molecule that is common in toll-like receptors (TLRs). It has been known that, among such toll-like receptors, TLR2 and TLR4 are able to recognize multiple proteins released from dead cells (Non Patent Literature 10). Thus, PECs prepared from wild-type (WT) mice, TLR2-deficient mice (TRL2 KO), and TLR4-deficient mice (TLR4 KO) were stimulated with recombinant TCTP, and the expression level of Cxcl1 mRNA was then measured. As a result, induction of Cxcl1 mRNA by TCTP was abolished in Tlr2-deficient PECs, but it was not abolished in Tlr4-deficient PECs (Figure 10(d)). In the present experiment, since induction of Cxcl1 mRNA by recombinant IL-1α in Tlr2-deficient PECs normally occurred, it was suggested that IL-1α be not associated with tumor proliferation by TLR2 (Figure 11(d)). Interaction between TCTP and TLR2 was confirmed by co-immunoprecipitation of epitope-tagged TCTP with TLR2 (Figure 11(e)). These results support the role of TLR2 as a signal receptor in induction of the expression of CXCL 1/2 via TCTP. Besides, as demonstrated by luciferase reporter assay, none of TLR3, TLR7 and TLR9 that emit signals via an MyD88 pathway were activated by recombinant TCTP (Figure 11(f)).

It is notable that SL4 tumors transplanted into Tlr2-deficient mice show proliferation retardation, compared with SL4 tumors transplanted into WT mice (Figure 11(g)). In addition, proliferation of IL-2-ss-TCTP tumors transplanted into Tlr2-deficient mice was also slow (Figure 11(h)). Moreover, O-vanillin as a selective TLR2 inhibitor suppressed proliferation of IL-2ss-TCTP tumors (Figure 11(i)). The aforementioned results show that continuous weak signals caused by tumor-derived TCTP (which are not transient strong signals caused by infection, etc.) are important for induction and maintenance of sufficiently developed TIME.

### 5. Effect of TCTP inhibition on tumor proliferation

Next, the influence of a TCTP inhibitory antibody and a TCTP inhibitor on proliferation of tumors was studied. Monoclonal antibodies (55F3, 44E1, and 51A9) reacting against a human TCTP peptide (SEQ ID NO: 25) were prepared. The peptide as set forth in SEQ ID NO: 25 consists of 20 residues on the C-terminal side of a human TCTP protein.

First, it was confirmed that the 55F3 antibody (55F3) has species crossing property to mouse TCTP (Figure 12(a)). When PECs were stimulated with a culture supernatant of SL4 cells containing 55F3 or control IgG, the expression of Cxcl1 mRNA from PECs stimulated with the culture supernatant of SL4 cells containing 55F3 was suppressed (Figure 12(b)). Subsequently, SL4 cells were transplanted into C57BL/6 mice via subcutaneous injection, and 55F3 or control IgG (200 µg/mouse) was then intraperitoneally administered to the mice every other day, starting from Day 1 after the transplantation, and the volume of the tumor was measured. As a result, the proliferation speed of SL4 tumor cells was decreased by administration of 55F3 (Figure 13(a)), and the amount of PMN-MDSCs in the TIME was decreased (Figure 6(b)). These results show that if the TCTP-CXCL1/2-MDSC pathway is inhibited, proliferation of tumors is suppressed.

Moreover, the effects of dihydroartemisinin (DHA) that is known to bind to TCTP and to promote decomposition of the TCTP in the proteasome were examined (Non Patent Literature 11). As shown in Figure 6(c), it was found that proliferation of SL4 tumors was inhibited by intraperitoneal administration of DHA. Meanwhile, the effect of the DHA to inhibit tumor proliferation was not observed in TCTP KO tumors (Figure 12(c)). Thus, it was demonstrated that TCTP is actually targeted in inhibition of tumor proliferation. The aforementioned results show that a TCTP inhibitory substance (TCTP antagonist) is effective as a cancer-treating agent.

The influence of a combination of inhibition of the function of TCTP with inhibition of PD-1 immune checkpoint on tumor proliferation was studied. As a PD-1 immune checkpoint antibody, an antibody of clone RMP1-14 (BioLegend) was used. SL4 cells were transplanted into C57BL/6 mice via subcutaneous injection, and thereafter, 55F3 or DHA was intraperitoneally transplanted into the mice every day, starting from Day 1 after the transplantation. Ten days after the transplantation, an anti-PD-1 monoclonal antibody was administered to the mice, and the tumor volume was then measured. As a result, it was confirmed that the effect of suppressing tumor proliferation is improved by the combined administration of 55F3 or DHA and the PD-1 antagonist antibody, compared with the case of single administration of 55F3 or DHA (Figures 13(d) and (e)).

### 6. Involvement of TCTP in human cancer

In order to study the role of TCTP in human cancer, the amount of a TCTP protein in the sera derived from human colorectal cancer (CRC) patients was measured. As with mouse models (Figure 2(b) and Figure 3(g)), the amount of the TCTP protein in the cancer patient-derived serum samples was larger than the amount of the TCTP protein in control samples (Figure 13(f)). In addition, when compared with normal large intestinal tissues, the amount of the TCTP protein in CRC tissues was large (Figure 13(g)). Moreover, an increase in the expression level of TCTP was correlated with progression of cancer (Figure 13(h)). An increase in the amount of the TCTP protein was confirmed in a neoplastic lesion, but was not confirmed in an interstitial area (Figure 12(d)). These results show that the expression of TCTP increases selectively in tumor cells. Further, when CRC tissues were stained with an antibody against CD15 that is a human PMN-MDSC marker, the expression level of a TCTP protein was positively correlated with the number of CD15⁺ cells (Figure 13(i)). These results suggest that a TCTP-OMN-MDSC pathway should also function in human cancer.

Furthermore, the Cancer Genome Atlas (TCGA)-derived data were analyzed. The TCTP mRNA levels, which were classified by the DNA copy numbers of CRC (colorectal cancer) patients (n = 376) obtained from TCGA database, are shown in Figure 13(j). The patients with Deep or Shallow each deleted 1 or 2 alleles of the TCTP gene, and the patients with Gain or Amplification each acquired 1 or more alleles of the TCTP gene. These analysis results show that amplification of the TCTP gene is observed in about 5% of the colorectal cancer patients, and that the expression level of TCTP mRNA is correlated with the copy number of the TCTP gene (Figure 13(j)). In addition, the TCTP expression level was negatively correlated with cytotoxic T cell or NK cell markers (i.e., CD8A, GZMB, PRF1, and CD69) (Figure 12(e)). This negative correlation was found also in terms of the cytolytic reactions of cytotoxic T cells and NK cells (each of which is defined as a geometric mean of GAMA mRNA and PRF 1 mRNA, respectively) (Figure 12(f)). Further, remarkably, the progression-free survival (PFS) of patients with an amplified TCTP gene was significantly low (Figure 13(k)).

Based on the aforementioned findings, the function of TCTP in tumor proliferation was summarized in Figure 13(l). TCTP is released from tumor dead cells and binds to a TLR2 receptor on M-MDSCs, so that it induces CXCL1/2 chemokine. It is concluded that such chemokine induces PMN-MDSCs to the TIME, attenuates antitumor immune response, and further promotes tumor growth.

### Industrial Applicability

The present invention provides a therapeutic drug and a therapeutic method for cancer, etc. Therefore, it is greatly expected that the present invention will be utilized in the medical field.

## Claims

1. An inhibitor of the accumulation of myeloid-derived suppressor cells (MDSCs) in the tumor microenvironment (TIME), wherein the inhibitor comprises, an active ingredient, a substance that suppresses or inhibits the function of an immunomodulator released from dead tumor cells.

2. The inhibitor according to claim 1, wherein the myeloid-derived suppressor cells are polymorphonuclear myeloid-derived suppressor cells (PMN-MDSCs).

3. The inhibitor according to claim 1 or claim 2, wherein the immunomodulator is TCTP (translationally controlled tumor protein).

4. The inhibitor according to claim 3, wherein the substance that suppresses or inhibits the function of TCTP is an antibody.

5. The inhibitor according to claim 3, wherein the substance that suppresses or inhibits the function of TCTP is dihydroartemisinin (DHA).

6. A therapeutic drug or composition for cancer, comprising, as an active ingredient, the inhibitor according to any one of claim 1 to claim 5.

7. The therapeutic drug or composition according to claim 6, wherein the cancer is colorectal cancer, malignant melanoma, or fibrosarcoma.

8. A method for diagnosing or auxiliarily diagnosing cancer, comprising measuring the amount of TCTP mRNA or TCTP protein that is present in a sample derived from a subject.

9. The method according to claim 8, wherein the sample is blood or tissue.

10. An antibody, which is **characterized in that** the amino acid sequences of CDRs (complementarity determining regions) 1 to 3 satisfy any of the following (A), (B) or (C), or an antigen-binding fragment thereof:
(A) the CDRs have:
heavy chain CDR1 comprising the amino acid sequence as set forth in SEQ ID NO: 1,
heavy chain CDR2 comprising the amino acid sequence as set forth in SEQ ID NO: 2,
heavy chain CDR3 comprising the amino acid sequence as set forth in SEQ ID NO: 3,
light chain CDR1 comprising the amino acid sequence as set forth in SEQ ID NO: 4,
light chain CDR2 comprising the amino acid sequence as set forth in SEQ ID NO: 5,
and
light chain CDR3 comprising the amino acid sequence as set forth in SEQ ID NO: 6,
(B) the CDRs have:
heavy chain CDR1 comprising the amino acid sequence as set forth in SEQ ID NO: 7,
heavy chain CDR2 comprising the amino acid sequence as set forth in SEQ ID NO: 8,
heavy chain CDR3 comprising the amino acid sequence as set forth in SEQ ID NO: 9,
light chain CDR1 comprising the amino acid sequence as set forth in SEQ ID NO: 10,
light chain CDR2 comprising the amino acid sequence as set forth in SEQ ID NO: 11,
and
light chain CDR3 comprising the amino acid sequence as set forth in SEQ ID NO: 12,
or
(C) the CDRs have:
heavy chain CDR1 comprising the amino acid sequence as set forth in SEQ ID NO: 13,
heavy chain CDR2 comprising the amino acid sequence as set forth in SEQ ID NO: 14,
heavy chain CDR3 comprising the amino acid sequence as set forth in SEQ ID NO: 15,
light chain CDR1 comprising the amino acid sequence as set forth in SEQ ID NO: 16,
light chain CDR2 comprising the amino acid sequence as set forth in SEQ ID NO: 17,
and
light chain CDR3 comprising the amino acid sequence as set forth in SEQ ID NO: 18.

11. An antibody that suppresses or inhibits the function of TCTP, wherein the antibody competitively inhibits the binding of the antibody according to claim 10 to TCTP, or an antigen-binding fragment thereof.

12. The antibody according to claim 10 or claim 11, which is **characterized in that** it is a humanized antibody, or an antigen-binding fragment thereof.

13. The antigen-binding fragment according to any one of claim 10 to claim 12, which is **characterized in that** it is Fab, Fab', F(ab')₂, Fv, a single chain antibody, scFv, an scFv dimer, or dsFv.
